# EUROPÄISCHE PATENTSCHRIFT

(11) **EP 3 773 332 B1**
(45) Veröffentlichungstag und Bekanntmachung des Hinweises auf die Patenterteilung: **07.06.2023**
(21) Anmeldenummer: 19717413.9
(22) Anmeldetag: 10.04.2019
(51) Int. Cl.: A61F 2/16, A61F 2/14, A61F 9/007

(54) **DUALFUNKTIONSINJEKTOR**
DUAL FUNCTION INJECTOR
INJECTEUR À DOUBLE FONCTION

(30) Priorität: 12.04.2018 CH 4672018
(43) Veröffentlichungstag der Anmeldung: 17.02.2021
(73) Patentinhaber: Medicel AG, 9423 Altenrhein (CH)
(72) Erfinder: DOCKHORN, Volker, 9423 Altenrhein (CH); GERMANN, Reto, 9423 Altenrhein (CH)
(74) Vertreter: Swisspat Riederer Hasler Patentanwälte AG
(86) Internationale Anmeldenummer: PCT/CH2019/050006
(87) Internationale Veröffentlichungsnummer: WO 2019/195951

(56) Entgegenhaltungen:
- WO-A1-2011/126144
- US-A1- 2009 112 223
- US-A1- 2014 257 317

## Beschreibung

### TECHNISCHES GEBIET DER ERFINDUNG

Das Gebiet der Erfindung umfasst einen Injektor, insbesondere einen Injektor zum Injizieren einer intraokularen Linse (IOL) in ein Auge oder zum Implantieren eines cornealen Endothelgewebes in ein Auge, gemäss dem Oberbegriff des Patentanspruchs 1.

### HINTERGRUND DER ERFINDUNG

Die in der Vergangenheit üblichen Injektoren zum Einsetzen von intraokularen Linsen (IOL) sind entweder in Spritzenform ausgeführt, welche einhändig betrieben werden können, oder sind mit Schraubgewinde ausgeführt, welche üblicherweise zweihändig betrieben sind. Abhängig davon, welche Technik ein Operateur erlernt hat bzw. bevorzugt, wird er einen Stossinjektor oder einen Schraubinjektor wählen.

Neben den zuvor genannten Stossinjektoren und Schraubinjektoren gibt es zudem Systeme, welche beide Funktionalitäten (d.h. die eines Stossinjektors und die eines Schraubinjektors) vereinen, d.h. dual sind, z.B. der IOL-Injektor DualTec^{™} bereitgestellt von OPHTEC BV. Auch auf dem Gebiet der Spritzen sind duale Systeme bekannt, z.B. in US 4,810,249, US 2009 112 223 oder WO 2011 126 144.

Dualfunktionale Systeme haben den Vorteil, dass es genügt, dem Operateur lediglich einen Injektor-Typ bzw. Spritzen-Typ zur Verfügung zu stellen (bzw. im Lager zu halten), denn es bleibt dem Operateur überlassen, in welcher Funktionalität er diesen verwendet. Insbesondere bei IOL-Systemen, in denen die IOL bereits im Injektor vorgeladen ist, können bei Verwendung eines Systems mit wahlweisein Stoss-/Schraubmechanismus Lagerhaltungs- und Beschaffungskosten für Linsenhersteller und Endanwender nahezu halbiert werden, da nur ein System auf Lager zu halten ist. Die bisher erhältlichen IOL-Injektoren mit Doppelfunktion haben jedoch einen erheblichen Mangel. Dieser Mangel besteht darin, dass das Gehäusedesign und insbesondere die für die Stossfunktion benötigte Griffplatte, welche bei Stossinjektoren verwendet wird, sich bei der Verwendung des Injektors in Schraubfunktion als äusserst hinderlich auswirkt, da der Injektor in Schraubfunktion anders als in Stossfunktion zweihändig verwendet wird und die Griffplatte hierbei im Weg ist.

Ein weiterer Nachteil von Injektoren, unabhängig davon, ob es sich um Stossinjektoren oder Schraubinjektoren handelt, ist (trotz gleichbleibender Konstruktion) die Variabilität des Kolbenendpunkts in Relation zum Düsenausgang. Wird ein elastischer Stempel, z.B. ein Silikonstempel, auf der Kolbenstange verwendet, ist eine besonders starke Variabilität zu erwarten. Dies liegt z.B. daran, dass die Länge der Kolbenstange nicht immer genau gleich ist. Insbesondere ist es bei Verwendung eines elastischen Stempels (wie z.B. eines Silikonstempels) auf der Kolbenspitze nicht möglich die Gesamtlänge des Kolbens genau zu bestimmen. Je nach verwendetem Linsenmaterial, Linsengeometrie, Dioptrie, viscoelastischer Lösung, Temperatur, Vorschub-Geschwindigkeit, u.s.w., wird der elastische Stempel mehr oder weniger stark zusammengestaucht (d.h. verkürzt) bzw. elongiert (d.h. verlängert), was je nach Bedingungen zu einer unterschiedlichen Gesamtlänge des Kolbens führt. Dies ist kritisch, da einerseits der Stempel stets das Ende der Injektordüse erreichen muss, damit die Linse in jedem Fall aus der Injektordüse herausgedrückt werden kann, zum anderen soll der Stempel nicht aus der Injektordüse herausgedrückt werden, da sich der zuvor in der Injektordüse zusammengestauchte Stempel dann soweit aufweitet, dass er kaum mehr in die Injektordüse zurückgezogen werden kann. Beim Herausziehen aus dem Auge, kann die Inzission durch den hervorstehenden, aufgeweiteten Silikonstempel aufreissen bzw. das Auge verletzt werden.

Im Weiteren erweist es sich bei beiden Injektor-Typen, unabhängig davon, ob es sich um Stossinjektoren oder Schraubinjektoren handelt, in der Praxis als schwierig, die Kolbenstange in eine oder mehrere aufeinander folgende vorgegebene Vorschubetappen sicher und unfehlbar vorzuschieben. Insbesondere ist es schwierig den Injektor so zu gestalten, dass die Vorschubetappe in einer bzw. jede der Vorschubetappen in je einer definierten Vorschubposition endet, welche gleichzeitig als Ausgangsposition dient, aus welcher die eigentliche Injektion oder gegebenenfalls die nächste Vorschubetappe folgen kann. Jede Vorschubposition sollte insbesondere einen genauen Kolbenstangenvorschub definieren, auf welche die Kolbenstangenspitze (d.h. das vordere Ende der Kolbenstange), insbesondere ein auf das vordere Ende der Kolbenstange aufgebrachter Stössel, genau einstellbar ist. Eine solche Vorschubposition kann zum Beispiel notwendig sein, um die sogenannten Haptiken der IOL definiert an die Optik anzulegen oder um ein Verrutschen der IOL aus der Ladekammer heraus durch den Silikonstempel zu blockieren.

Es gibt IOL-Systeme mit einem Schnapper (Federteil), welcher die Kolbenspitze bzw. den darauf sitzenden Stössel vor dem Herausfallen aus dem Injektorgehäuse bewahrt. Beispiele hierfür sind die im Handel erhältlichen Injektor-Systeme VISCOJECT^{™}, NAVIJECT^{™} und ACCUJECT^{™} der Medicel AG. Eine Zusätzliche Funktion dieses Schnappers ist es, eine zweite Vorschubposition des Kolbens zu sichern. Dabei springt der Schnapper beim erstmaligen Vorschieben aus einem ersten Loch im Injektorgehäuse, schiebt nach vorne und rastet in einem zweiten Loch im Injektorgehäuse ein. Diese zweite Schnappung ist spürbar und/oder mit eine kleinen "Klick" verbunden. Trotzdem wird diese zweite Kolbenposition in der Praxis oftmals missachtet bzw. übersehen und deshalb überfahren. Die Problematik besteht darin, dass der durch die Schnappung erzeugte Widerstand so stark gestaltet werden müsste, dass ein unabsichtliches Überfahren vermieden werden könnte. Allerdings würde ein so starkes Einschnappen bzw. ein so starker Wiederstand auch dazu führen, dass ein Herausdrücken aus dieser Vorschubposition beim weiteren Vorschub des Injektors eine schlagartige Bewegung erzeugen würde (nämlich in dem Moment, indem der Wiederstand überwunden ist), so dass die IOL unkontrolliert und schlagartig über einen grösseren Bereich vorbewegt wird. Dies wiederspricht jedoch der Anforderung, dass eine IOL zu jedem Zeitpunkt kontrolliert vorgeschoben werden muss.

Die genannten Probleme hinsichtlich Variabilität des Kolbenendpunktes und der Vermeidung des Überfahrens der Vorschubposition treten auch bei Injektoren mit Doppelfunktion auf.

### AUFGABE

Es ist eine Aufgabe der vorliegenden Erfindung, einen alternativen Injektor bereitzustellen. Insbesondere ist es ein Ziel, einen Injektor zu entwickeln, der möglichst viele oder alle der genannten Probleme löst.

Insbesondere soll ein Injektor entwickelt werden, der unter Berücksichtigung der oben genannten Anforderungen wahlweise als Stoss- oder Schraubinjektor (d.h. Push- oder Screw-Injektor) verwendet werden kann. Insbesondere sollten Strukturen, welche für eine Stossfunktion relevant sind bei Gebrauch des Injektors als Schraubinjektor nicht hinderlich sein und umgekehrt sollten Strukturen, welche für eine Schraubfunktion relevant sind bei Gebrauch des Injektors als Stossinjektor nicht hinderlich sein.

Weitere ist es Aufgabe, ein derartiges System zu entwickeln, welches ein wiederholtes Wechseln vom Stoss- in den Schraubbetrieb und umgekehrt zulässt. Es soll also eine reversible Lösung gefunden werden. Zudem soll das System genügend Stabilität bieten, um eine interokulare Linse in ein Auge injizieren zu können, und gleichzeitig auch kostengünstig sein.

Es wird zudem angestrebt die Variabilität des Kolbenendpunkts in Relation zum Düsenausgang zu minimieren, insbesondere bei Verwendung eines elastischen Stempels auf der Kolbenstange. Diese Lösung soll für Stossinjektoren und Schraubinjektoren und insbesondere auch Dualinjektoren, d.h. Injektoren mit Stoss- und Schraubfunktion, anwendbar sein.

Es wird angestrebt die Einstellung des Kolbens auf Vorschubetappen zu verbessern, insbesondere so zu verbessern, dass vorgegebene Vorschubetappen sensorisch (z.B. visuell, auditiv und/oder taktil) vom Operateur unfehlbar bemerkbar sind und der Kolben darauf genau einstellbar ist, ohne dass beim weiteren Vorschub aus einer Vorschubposition heraus die Linse unkontrolliert fortbewegt wird. Dies ist insbesondere bei Vorschubpositionen von Bedeutung, aus welchen mit dem nächsten Vorschubschritt die Kolbenstangenspitze mit der Linse in Kontakt treten soll oder bei welchen die Kolbenstange schon mit der Linse in Kontakt steht.

### ZUSAMMENFASSUNG DER ERFINDUNG

Die Erfindung bezieht sich weiter auf einen Injektor (z.B. einen Injektor zum Ausstossen einer intraokularen Linse zwecks Injizierens dieser in ein Auge oder einen Injektor zum Implantieren eines cornealen Endothelgewebes) beinhaltend einen länglichen Injektorkörper mit Kolbengang, in welchem eine Injektorkolbenstange, welche ein Schraubgewinde aufweist, longitudinal (in Achsrichtung der Injektorkolbenstange) verschiebbar geführt ist, wobei der Injektor zur Verschiebung der Injektorkolbenstange mit zwei Betriebsmodi ausgestattet ist, zwischen welchen Modi umgeschaltet werden kann, wobei der erste Betriebsmodus einen Stossbetrieb und der zweite Betriebsmodus einen Schraubbetrieb definiert und wobei sich der Injektor dadurch auszeichnet, dass der Injektorkörper zumindest einen ein- und ausklappbaren (bzw. ein- und ausschwenkbaren) Flügel bzw. Flügelgriff aufweist, wobei durch die ausgeklappte Position der Betriebsmodus auf Stossbetrieb und durch die eingeklappte Position der Betriebsmodus auf Schraubbetrieb gesetzt ist. Dadurch kann der Injektor als Stossinjektor oder als Schraubinjektor verwendet werden. Der zumindest eine Flügel bzw. Flügelgriff ist vorzugsweise längsseitig am Injektorkörper angelegt.

Ist der Flügelgriff ausgeklappt, bedeutet dies, dass der Flügel mit seinem Griffbereich in greifbarer Stellung vom Injektorkörper absteht und vorzugsweise vorderseitig (d.h. von der Düsenseite des Injektors her) greifbar ist, um zum Beispiel per einhändigem Dreifingergriff (das heisst z.B. je ein Finger auf einem Flügelgriff und einen dritten Finger (vorzugsweise den Daumen) am hinteren Ende der Kolbenstange) die Injektorkolbenstange gegen den Flügelgriff anzustossen und dabei Richtung Düse zu stossen.

Die Flügelgriffe stehen in aufgeklappter Stellung flügelartig vom Injektorkörper ab. Die Flügelgriffe sind in aufgeklappter Stellung zweckmässigerweise gegen ein weiteres Umklappen bzw. ein Einklappen nach hinten, d. h. zum proximalen Ende des Injektors hin, blockiert. Griffbereiche am Flügelgriff sind zweckmässigerweise an jener Seite des Flügelgriffs angelegt, welche am aufgeklappten Flügelgriff zur Injektordüse hin gewandt ist. Die Flügelgriffe sind zweckmässigerweise derart ausgelegt, dass in aufgeklappter Stellung deren Griffbereiche als Gegendruckflächen zu einer Druckfläche am proximalen Ende der Injektorkolbenstange dient. Insbesondere sind die Flügelgriffe derart ausgelegt, dass diese in aufgeklappter Stellung, d.h. in Stossfunktionsstellung, zumindest einer Druckkraft auf die Griffbereiche widerstehen, welche der auf das proximale Ende der Injektorkolbenstange aufgebrachten Stosskraft entspricht. Insbesondere sollten die aufgeklappten Flügelgriffe einer Kraft von mindestens 10 Newton, bevorzugt mindestens 20 N und weiter bevorzugt mindestens 30 N standhalten.

Zweckmässigerweise ist der mindestens eine ein- und ausklappbare Flügelgriff in Bezug auf den Injektorkörper von vorne her ausklappbar und nach vorne hin wieder einklappbar. Der zumindest eine Flügelgriff ist insbesondere derart angelegt, dass sich dieser nach vorne, das heisst mittels einer Klappbewegung zur Injektordüse hin, einklappen lässt, und nach hinten, das heisst mittels einer Klappbewegung zum proximalen Injektorende hin, ausklappen lässt.

Ist der Flügelgriff eingeklappt, bedeutet dies, dass der Flügel mit seinem Griffbereich am Injektorkörper längsseitig anliegt und vorzugsweise in der Seitenwand des Injektors versenkt ist, sodass Injektorkörperaussenwand und eingeklappter Flügel eine relativ gerade bzw. homogene Fläche bilden, damit der Injektorkörper hindernisfrei mit einer ersten Hand (z.B. wie ein Schreibstift) gehalten werden kann, um zum Beispiel mit der zweiten Hand die Injektorkolbenstange Richtung Düse zu schrauben.

Der Injektor zeichnet sich vorteilhafterweise dadurch aus, dass abhängig von der Stellung des zumindest einen Flügelgriffs der Injektor als Stossinjektor oder als Schraubinjektor funktioniert (duale Injektionsfunktion). In ausgeklappter Position des zumindest einen Flügelgriffs (d.h. im Betriebsmodus Stossbetrieb) stehen die Flügelgriffe vom Injektorkörper ab. Dabei bilden die Flügelgriffe bevorzugt eine zur Längserstreckung des Injektorkörpers im Wesentlichen orthogonal angeordnete Griffflächen. Dadurch kann zwecks Stossbetrieb der Injektor z.B. mit einem oder mehreren Fingern einer Hand an den Flügelgriffen gefasst werden, während mit dem Daumen derselben Hand der Kolben an seinem Ende gehalten und ggf. gestossen wird. In eingeklappter Position des zumindest einen Flügelgriffs (d.h. im Betriebsmodus Schraubbetrieb) ist der zumindest eine Flügelgriff gegen den Injektorkörper eingeklappt. Dabei bildet der Injektor zusammen mit dem zumindest einen Flügelgriff im Wesentlichen einen gleichförmig longitudinalen Stiel (ohne seitlich abstehende Fortsätze). Dabei ist der zumindest eine Flügelgriff vorzugsweise in die Längsseite des Injektorkörper integriert. Dadurch kann zwecks Schraubbetrieb der Injektorkörper längsseitig mit den Fingern der ersten Hand problemlos gefasst werden, während mit der zweiten Hand der Kolben in Schraubdrehung versetzt wird.

Der Injektor zeichnet sich insbesondere dadurch aus, dass der Flügelgriff derart ausgeführt ist, dass das Ein- und das Ausklappen des Flügelgriffs wiederholbar ist. Somit kann prinzipiell beliebig von einem Modus in den anderen gewechselt werden.

Der Injektor zeichnet sich vorzugsweise dadurch aus, dass der Injektorkörper zumindest einen verschieblichen, insbesondere einen ein- und ausschiebbaren Gewindesteg aufweist. Der zumindest eine Gewindesteg kann zweckmässigerweise in den Kolbengang hinein und heraus geschoben werden. Einerseits ist der Gewindesteg soweit in den Kolbengang einschiebbar, dass der Gewindesteg als Innengewinde bezüglich des Schraubgewindes der Kolbenstange wirkt und eine Linse durch Schraubbewegung der Kolbenstange ausgestossen werden kann. In dieser Schraubfunktion des Injektors läuft das Schraubgewinde der Kolbenstange im Gewindesteg des Injektorkörpers bzw. kann das Schraubgewinde der Kolbenstange am Gewindesteg des Injektorkörpers in den Kolbengang bzw. den Injektorkörper eingeschraubt werden. Andererseits ist der Gewindesteg soweit aus dem Kolbengang ausschiebbar bzw. von der Kolbenpassage wegziehbar, dass der Gewindesteg nicht mehr als Innengewinde bezüglich des Schraubgewindes der Kolbenstange wirkt (und den Durchgang der Kolbenstange auch sonst nicht behindert) und somit eine Linse oder Implantat durch einfache Stossbewegung der Kolbenstange (d.h. durch Stossbewegung auch ohne Drehanteil) ausgestossen werden kann.

Besonders vorteilhaft ist, dass der zumindest eine Flügelgriff und der zumindest eine Gewindesteg kooperativ in Verbindung stehen, sodass beim Ein- oder Ausklappen (bzw. Ein- oder Ausschwenken) des zumindest einen Flügelgriffs der zumindest eine Gewindesteg ebenfalls ein- und ausgeschoben wird. Bevorzugterweise wird beim Ausklappen des Flügelgriffs (27, 28) der Gewindesteg aus der Kolbenpassage ausgeschoben und beim Einklappen des Flügelgriffs in die Kolbenpassage eingeschoben.

Insbesondere sind der zumindest eine Flügelgriff und der zumindest eine Gewindesteg in Wirkverbindung angeordnet, sodass durch Einklappen des zumindest einen Flügelgriffs der zumindest eine Gewindesteg in eine erste Position führbar ist, in welcher der zumindest eine Gewindesteg ein Gegengewinde für das Schraubgewinde der Injektorkolbenstange bildet (d.h. zwecks Schraubbetrieb) und durch Ausklappen des zumindest einen Flügelgriffs der zumindest eine Gewindesteg in eine zweite Position führbar ist, in welcher der zumindest eine Gewindesteg kein Gegengewinde für das Schraubgewinde der Injektorkolbenstange bildet (d.h. zwecks Stossbetrieb).

Flügelgriff und Gewinde stehen vorzugsweise derart miteinander in Verbindung, dass bei eingeklapptem Flügel, dieser nicht durch mechanischen Druck auf das Gewinde selbstständig aufklappt.

Insbesondere stehen Flügelgriff und Gewindesteg derart miteinander in Wirkverbindung, dass bei eingeklapptem Flügelgriff durch mechanischen Druck der Kolbenstange auf den Gewindesteg, wie er beim Einschrauben der Kolbenstange entsteht, der Flügelgriff nicht bzw. nicht selbständig aufklappen kann. Oder anders ausgedrückt, Flügelgriff und Gewindesteg stehen insbesondere derart miteinander in Wirkverbindung, dass bei eingeklapptem Flügelgriff der Flügelgriff auch dann in eingeklappter Position bleibt, wenn beim Einschrauben der Kolbenstange die Kolbenstange mechanischen Druck auf den Gewindesteg ausübt. Ein Aufklappen des Flügelgriffs ist vorzugsweise lediglich durch aktives manuelles Aufklappen des Flügelgriffs (d.h. somit von Ausserhalb des Injektorkörpers) erzielbar.

Beinhaltet der Injektor zwei Flügel und somit in Wirkverbindung mit jeweils einem Flügel ein Gewindesteg, dann bilden die Gewindestege bei eingeklappten Flügeln gemeinsam ein Innengewinde für das Schraubgewinde der Kolbenstange. Bei aufgeklappten Flügeln sind die Gewindestege jedoch weiter voneinander beabstandet als bei eingeklappten Flügeln und bilden daher kein Innengewinde für das Schraubgewinde der Kolbenstange sondern erlauben vielmehr ein Durchstossen der Kolbenstange durch den Kolbengang ohne Schraubdrehung der Kolbenstange.

Der Injektor zeichnet sich vorteilhafterweise dadurch aus, dass der Injektorköper derart ausgeführt ist, dass abhängig von der Stellung des zumindest einen Flügelgriffs und insbesondere von der Stellung des zumindest einen Gewindestegs der Injektor als Stossinjektor oder als Schraubinjektor funktioniert (duale Injektionsfunktion).

Der zumindest eine Flügelgriff und der zumindest eine Gewindesteg sind zweckmässigerweise gemeinsam in zumindest einer Dualfunktionsgruppe integriert. Die Elemente der zumindest einen Dualfunktionsgruppe sind vorzugsweise längsseitig am Injektorkörper angelegt.

Bevorzugterweise sind der zumindest eine Flügelgriff und der zumindest eine Gewindesteg gelenkig mit dem Injektorgehäuse verbunden, was insbesondere das Ein- oder Ausklappen bzw. Ein- oder Ausschwenken von Flügelgriff und Gewindesteg ermöglicht.

Die duale Injektionsfunktion zeichnet sich vorzugsweise dadurch aus, dass der Injektorköper derart ausgeführt ist, dass abhängig von der Stellung des zumindest einen Flügelgriffs bzw. des zumindest einen Gewindestegs der Injektor als Stossinjektor oder als Schraubinjektor funktioniert bzw. funktionieren kann.

Insbesondere ist hierin ein Dualinjektor (Injektor mit Dualfunktion) vorgestellt und beschrieben, welcher erfindungsgemäss mit verstellbaren Dualfunktionsgruppen zur Umstellung von der Stossfunktion in die Schraubfunktion ausgestattet ist. Die Dualfunktionsgruppen sind insbesondere versstellbar, indem der zumindest eine Flügelgriff ein- und ausklappbar, bzw. ein- und ausschwenkbar ausgeführt ist.

Vorteilhafterweise zeichnet sich der Injektor dadurch aus, dass der zumindest eine ein- und ausklappbare Flügelgriff eine Drehachse aufweist, um welche der Flügelgriff ein- und ausklappbar ist.

Zweckmässigerweise stehen Flügelgriff und Gewindesteg über ein Druckmittel miteinander in Wirkverbindung, welches Druckmittel am Flügelgriff exzentrisch zur Drehachse des Flügelgriffs ausgebildet ist. Ein derartiges Druckmittel wird im Weiteren auch Exzenter genannt.

Das Druckmittel bzw. der Exzenter, welches bzw. welcher exzentrisch zur Drehachse angeordnet ist, hält bei eingeklapptem Flügelgriff den Gewindesteg in einer ersten Position, in welcher der zumindest eine Gewindesteg ein Gegengewinde für das Schraubgewinde der Injektorkolbenstange bildet, sodass die Injektorkolbenstange schraubbar ist, und hält bei ausgeklapptem Flügelgriff den Gewindesteg in einer zweiten Position, in welcher der zumindest eine Gewindesteg kein Gegengewinde für das Schraubgewinde der Injektorkolbenstange bildet, sodass die Injektorkolbenstange stossbar ist.

Der Exzenter (d.h. das Druckmittel) setzt beim Einklappen des Flügelgriffs den Gewindesteg an die Führungspassage der Injektorkolbenstang im Injektorkolbengang heran und zieht beim Ausklappen des Flügelgriffs den Gewindesteg von der Führungspassage der Injektorkolbenstange weg, sodass im ersten Fall der zumindest eine Gewindesteg ein Gegengewinde für das Schraubgewinde der Injektorkolbenstange bildet und im zweiten Fall der zumindest eine Gewindesteg soweit von der Führungspassage entfernt ist, dass der zumindest eine Gewindesteg kein Gegengewinde für das Schraubgewinde der Injektorkolbenstange bildet.

Beinhaltet der Injektor zwei Flügel und somit an jedem Flügel einen Exzenter (Druckmittel), dann sind die Exzenter (Druckmittel) bei aufgeklappten Flügeln vorzugsweise weiter voneinander beabstandet als bei eingeklappten Flügeln, was bewirkt dass gleichzeitig bei aufgeklappten Flügeln die Gewindestege weiter voneinander beabstandet sind als bei eingeklappten Flügeln.

Zweckmässigerweise ist der zumindest eine Gewindesteg an zumindest einer beweglichen Halterung ausgebildet, welche derart beweglich ist, dass der Abstand des Gewindestegs zum Kolbengang veränderbar ist, insbesondere durch das Verstellen (d.h. Ausklappen und Einklappen) der Flügelposition. Zweckmässigerweise setzt die Halterung am Injektorkörper an bzw. ist am Injektorkörper befestigt.

Der Abstand des Gewindestegs zum Kolbengang (bzw. zur Kolbenstangenpassage) bzw. dessen Mitte ist dadurch veränderbar, dass der Gewindesteg in den Kolbengang bzw. in die Kolbenstangenpassage eingeschoben oder aus dem Kolbengang bzw. aus der Kolbenstangenpassage herausgeschoben bzw. herausgezogen werden kann.

Dadurch dass Flügelgriff und Gewindesteg in kooperativer Wirkverbindung stehen, wird durch das Einklappen des Flügelgriffs der Gewindesteg aus dem Kolbengang herausgezogen und durch das Ausklappen des Flügelgriffs der Gewindesteg in den Kolbengang hineingeschoben.

Die zumindest eine bewegliche Halterung kann als Gabel mit einem inneren Schenkel und einem äusseren Schenkel ausgebildet sein. Der zumindest eine Gewindesteg ist kolbengangseitig an der Halterung ausgebildet. Vorzugsweise ist der zumindest eine Gewindesteg am innerer Schenkel (d.h. am Schenkel, der weiter in den Kolbengang hineinreicht) gabelaussenseitig (d.h. kolbengangseitig) ausgebildet, sodass der Gewindesteg in seiner erster Position ein Innengewinde für das Schraubgewinde der Injektorkolbenstange bildet.

Im Weiteren kann die bewegliche Halterung, insbesondere die Gabel bzw. die Schenkel der Gabel (welche eine Art (offenes) Langloch bilden) den Exzenter (d.h. das Druckmittel) fassen bzw. umfassen, insbesondere in der ersten Position des zumindest einen Gewindestegs und der zweiten Position des zumindest einen Gewindestegs sowie in den dazwischen liegenden Positionen.

Beim Ein- und Ausklappen des zumindest einen Flügels um seine Drehhachse, bewegt sich der Exzenter (d.h. das Druckmittel) ebenfalls um die Drehachse des Flügels und nimmt dabei die Gabel durch schieben des äusseren Schenkels oder inneren Schenkels mit, wobei beim Einklappen des Flügels die Gabel am inneren Schenkel und damit der Gewindesteg eingeschoben wird und beim Ausklappen des Flügels die Gabel am äussere Schenkel und damit der Gewindesteg ausgeschoben wird.

Zweckmässigerweise ist der zumindest eine Flügelgriff längsseitig am Injektorkörper ein- und ausklappbar befestigt.

Es ist vorteilhaft, dass der mindestens eine ein- und ausklappbare Flügelgriff als Doppelflügelgriff ausgeführt ist, mit einem ersten Flügel und einem zweiten Flügel, vorzugsweise dass der erste Flügel und der zweite Flügel einander gegenüberliegend (auf gleicher Injektorerstreckung) längsseitig am Injektorkörper ein- und ausklappbar befestigt sind.

Vorteilhafterweise sind die Ein- und Ausklappbewegung der zwei Flügel synchronisiert, z.B. über eine Verzahnung. Die Verzahnung der zwei Flügel kann z.B. durch zwei Zahnräder oder zwei Teilzahnränder erzeugt werden, welche an und gegenüber den Flügeln fixiert sind. Vorzugsweise entsprechen die Drehachsen der Zahnräder den Drehachsen der jeweiligen Flügel.

Bevorzugterweise ist die Injektorkolbenstange rückseitig (d.h. an ihrem proximalen Ende) mit einem Betätigungselement ausgeführt, welches zur manuellen Bedienung des Kolbens, z.B. zum manuellen Drücken oder Drehen bzw. Schrauben des Kolbens, dient.

Die Anforderung, einen Injektor wahlweise als Stoss- oder Schraubinjektor (der Doppelfunktionsausführung eines Erfindungsgemässen Injektors) verwenden zu können, ist besonders vorteilhaft dadurch erfüllt, dass beim erfindungsgemässen Injektor das Gehäuse zweckmässigerweise derart ausgebildet ist, dass die Gehäuseform dem gewünschten Betriebsmodus (d.h. der Stossfunktion oder der Schraubfunktion) angepasst werden kann bzw. dass der Betriebsmodus am Injektor eingestellt werden kann.

Jeder der hierin vorgestellten Injektoren beinhaltet vorteilhafterweise einen länglichen Injektorkörper mit einem vorderen Ende des Injektorkörpers und einem hinteren Ende des Injektorkörpers. Im Injektorkörper bzw. in einem Gang des Injektorkörpers, welcher vom vorderen Ende des Injektorkörpers zum hinteren Ende des Injektorkörpers führt, ist zweckmässigerweise eine Injektorkolbenstange (vom hinteren Ende des Injektorkörpers her zum vorderen Ende des Injektorkörpers hin) longitudinal (bzw. axial) verschiebbar geführt. Im Injektorkörper ist vorzugsweise entweder eine Ladekammer für eine Linse enthalten oder eine Aussparung zur Aufnahme einer Ladevorrichtung mit Ladekammer für eine Linse vorgesehen. Die Ladekammer oder die Aussparung zur Aufnahme der Ladevorrichtung ist zweckmässigerweise derart ausgestaltet, dass die Injektorkolbenstange durch diese hindurch zum vorderen Ende des Injektorkörpers hin vorschiebbar ist.

Insbesondere ist die Aussparung derart ausgestaltet, dass die Injektorkolbenstange durch diese hindurch und gegebenenfalls durch eine in die Aussparung eingelegte Ladekammer hindurch zum vorderen Ende des Injektorkörpes hin vorschiebbar ist.

Zweckmässigerweise ist am vorderen Ende des Injektorkörpers eine Injektordüse vorgesehen, in Richtung welcher die Injektorkolbenstange vorschiebbar ist.

Zweckmässig ist, dass in der Aussparung eine Ladevorrichtung angeordnet ist oder angeordnet werden kann. Die Ladevorrichtung beinhaltet insbesondere eine Ladekammer für eine Linse, ein zu implantierendes corneales Endothelgewebe oder irgendein anderes Implantat, dass in das Auge implantiert werden soll. Düse und Ladekammer können wahlweise aus einem Teil oder in zwei separaten Teilen gefertigt sein. Bei einteiliger Fertigung ist eine Aussparung nicht notwendig.

In einer bevorzugten Ausführung des Injektors, zeichnet sich der Injektor dadurch aus, dass (a) der Injektorkörper zumindest einen ein- und ausklappbaren Flügelgriff aufweist, wobei durch die ausgeklappte Position der Betriebsmodus auf Stossbetrieb und durch die eingeklappte Position der Betriebsmodus auf Schraubbetrieb gesetzt ist, und (b) der Injektorkörper zumindest einen Gewindesteg aufweist, welcher im Schraubbetrieb ein Innengewinde für das Schraubgewinde der Kolbenstange bildet und welcher im Stossbetrieb nicht mit dem Schraubgewinde der Kolbenstange zusammenwirkt, z.B. dadurch dass im Stossbetrieb das Innengewinde gegenüber der Kolbenstangenachse radial weiter nach aussen versetzt ist als im Schraubbetrieb, wobei (c) vorzugsweise der zumindest eine Flügelgriff um eine Drehachse ein- und ausklappbar ist, (d) vorzugsweise der zumindest eine Flügelgriff einen Exzenter (auch Druckmittel genannt) beinhaltet, welcher exzentrisch zur Drehachse angeordnet ist, (e) vorzugsweise der Gewindesteg auf einer beweglichen Halterung ausgebildet ist, und/oder (f) vorzugsweise der Exzenter in die Halterung eingreift, sodass beim Ein- bzw. Ausklappen des Flügelgriffs der Gewindesteg durch Einwirkung des Exzenters auf die Halterung von der Kolbenstangenachse weggeschoben bzw. zur Kolbenstangenachse hingeschoben wird.

Die Erfindung bezieht sich im Weiteren auf einen Injektor (z.B. einen Injektor zum Ausstossen einer intraokularen Linse zwecks Injizierens dieser in ein Auge oder einen Injektor zum Implantieren eines cornealen Endothelgewebes) beinhaltend einen länglichen Injektorkörper, in welchem eine Injektorkolbenstange longitudinal (bzw. axial) verschiebbar geführt ist, wobei sich der Injektor dadurch auszeichnet oder zusätzlich dadurch auszeichnet, dass die Injektorkolbenstange ein erstes Anschlagelement beinhaltet. Ein derartiges Anschlagelement wird vorteilhafterweise an einem Injektor mit Dualfunktion wie oben beschrieben verwirklicht.

Das Anschlagelement zeichnet sich vorteilhafterweise dadurch aus, dass es ein verschiebbares Anschlagelement ist. Das Anschlagelement ist insbesondere auf der Kolbenstange axial (bzw. longitudinal, d.h. der Kolbenstange entlang) verschiebbar angeordnet. Das Anschlagelement umfasst zweckmässigerweise die Kolbenstange. Das Anschlagselement ist z.B. als anliegender Ring ausgeführt.

Das Anschlagelement kann als komprimierbares oder deformierbares Anschlagelement (d.h. Soft-Anschlag) ausgestaltet sein. Das Anschlagelement ist z.B. aus einem elastischen bzw. deformierbaren oder komprimierbaren Material, insbesondere aus Silikon oder TPE (thermoplastisches Elastomer), gefertigt. Das komprimierbare Anschlagelement ist vorzugsweise auf der Injektorkolbenstange verschiebbar angeordnet.

Vorzugsweise ist das Anschlagelement auf der Kolbenstange axial (bzw. longitudinal), d.h. der Kolbenstange entlang, verschiebbar angeordnet und deformierbar oder komprimierbar (insbesondere zumindest in axialer Richtung zur Kolbenstange komprimierbar) ausgeführt.

Die Injektorkolbenstange ist rückseitig zweckmässigerweise mit einem Betätigungselement das auch Anschlagsstütze genannt werden kann ausgestattet. Das Betätigungselement dient insbesondere dem manuellen Vorwärtsbewegen des Kolbens im Injektorkörper durch manuelles Stossen oder manuelles Drehen des Kolbens. Das Betätigungselement wirkt gegebenenfalls weiter als Rückhalt für das Anschlagelement (d.h. als Anschlagsstütze). Das Betätigungselement kann neben dem ersten Anschlag, welcher verschiebbar und/oder komprimierbar ist, als zweiter Anschlag dienen. Das Betätigungselement, d.h. der zweite Anschlag, ist in Bezug auf die Kolbenstange fixiert, d.h. nicht verschiebbar, und unter normalen Anwendungsbedingungen im erfindungsgemässen Injektor zumindest in longitudinaler Kolbenausrichtung nicht komprimierbar (das Betätigungselement wirkt somit als sog. harter Anschlag).

Vorzugsweise ist das Betätigungselement bzw. die Anschlagsstütze integraler Teil der Injektorkolbenstange bzw. auf der Injektorkolbenstange ausgebildet oder befestigt. Vorzugsweise ist das Betätigungselement am proximalen Ende der Injektorkolbenstange angelegt. Das Betätigungselement ist zweckmässigerweise mit Bezug auf die Injektorkolbenstange ortsfest angelegt.

Wahlweise kann das Betätigungselement bzw. die Anschlagsstütze eine Rampe beinhalten. Vorzugsweise ist diese im Übergang vom Kolbenstangenschaft zum Betätigungselement bzw. zur Anschlagsstütze hin als konische oder keilartige Vergrösserung oder Verdickung des Schaftes ausgebildet.

Auch bei der genannten Ausführung mit erstem Anschlagelement kann der Injektorkörper zumindest einen ein- und ausklappbaren (bzw. ein- und ausschwenkbaren) und/oder ein- und ausschiebbaren Flügelgriff aufweisen. Dies hat den Vorteil, dass sobald bzw. wenn der Injektor zum Einstossen der Linse oder des cornealen Endotheltransplantats verwendet wird (d.h. als Stossinjektor), der oder die Flügelgriffe in Funktionsstellung gebracht werden können und zu anderen Zeiten bzw. anderem Zwecke der oder die Flügelgriffe platzsparend verstaut sein können.

Es ist bevorzugt, dass die Injektorkolbenstange ein Schraubgewinde aufweist und der Injektorkörper zumindest einen verschieblichen (bzw. ein- und ausschiebbaren) Gewindesteg aufweist. Ein derartiger Injektor kann als Schraubinjektor verwendet werden, wobei gegebenenfalls der oder die Gewindestege in Funktionsstellung gebracht werden können, indem der oder die Gewindestege seitlich in den Kolbengang hineingesteckt werden, sobald bzw. wenn der Injektor zum Einbringen der Linse verwendet werden soll (d.h. als Schraubinjektor) und zu anderen Zeiten bzw. anderem Zweck des Injektors der oder die Gewindestege aus dem Kolbengang entfernt sein können.

Es ist vorteilhaft, dass zwecks dualer Injektionsfunktion der zumindest eine Flügelgriff und der zumindest eine Gewindesteg kooperativ miteinander in Verbindung stehen, sodass zweckmässigerweise beim Einklappen des zumindest einen Flügelgriffs der zumindest eine Gewindesteg vom Stossmodus in den Schraubmodus (d.h. eine aktive Position, in welcher der Gewindesteg mit dem Schraubgewinde der Injektorkolbenstange kooperiert) geführt wird bzw. sodass zweckmässigerweise beim Ausklappen des zumindest einen Flügelgriffs der zumindest eine Gewindesteg vom Schraubmodus in den Stossmodus (d.h. eine inaktive Position, in welcher der Gewindesteg nicht mit dem Schraubgewinde der Injektorkolbenstange kooperiert) geführt wird.

Zusätzliche Vorteile der vorliegenden Erfindung ergeben sich aus der nachfolgenden Beschreibung.

### KURZBESCHREIBUNG DER FIGUREN

Weitere Vorteile und Merkmale der Erfindung ergeben sich aus der nachfolgend detaillierten Beschreibung von Ausführungsbeispielen der Erfindung unter Bezugnahme auf schematische Darstellungen. Es zeigen in nicht massstabsgetreuer, schematischer Darstellung:
- Figur 1:: einen erfindungsgemässen Dualinjektor mit eingeklappten Flügeln in perspektivischer Ansicht;
- Figur 2:: einen erfindungsgemässen Dualinjektor mit ausgeklappten Flügeln in einer perspektivischer Ansicht;
- Figur 3:: einen erfindungsgemässen Dualinjektor mit geöffnetem Gehäuse;
- Figur 4:: einen erfindungsgemässen Dualinjektor, teilweise auseinandergenommen;
- Figur 5:: Schnittbild des hinteren Endes des Injektors bei aufgeklappten Flügeln;
- Figur 6:: Schnittbild des hinteren Endes des Injektors bei aufgeklappten Flügeln mit eingesetzter Kolbenstange;
- Figur 7:: Schnittbild des hinteren Endes des Injektors bei eingeklappten Flügeln;
- Figur 8:: Schnittbild des hinteren Endes des Injektors bei eingeklappten Flügeln mit eingesetzter Kolbenstange;
- Figur 9:: Schnittbild des hinteren Endes des Injektors bei eingeklappten Flügeln mit eingesetzter Kolbenstange, wobei Drehpunkt der Flügel und Position der Exzenter markiert sind;
- Figur 10:: linker Flügel in perspektivischer Ansicht;
- Figur 11:: linker Flügel in weiterer perspektivischer Ansicht;
- Figur 12:: linker Flügel in weiterer perspektivischer Ansicht;
- Figur 13:: einen Dualinjektor mit seiner Kolbenstange in einer ersten Vorschubposition (erste Vorschubposition, Ausgangsposition);
- Figur 14:: einen Dualinjektor mit seiner Kolbenstange in einer zweiten Vorschubposition (zweite Vorschubposition);
- Figur 15: einen Dualinjektor mit seiner Kolbenstange in einer dritten Vorschubposition (dritte Vorschubposition);
- Figur 16: einen Dualinjektor mit seiner Kolbenstange in einer vierten Vorschubposition (Reserveposition);
- Figur 17:: einen Dualinjektor mit seiner Kolbenstange in einer ersten Vorschubposition (alternative Ausgangsposition);
- Figur 18:: ein Stossinjektor mit Rampe und Schlitten mit seiner Kolbenstange in einer ersten Vorschubposition (erste Vorschubposition).

### DETAILIERTE BESCHREIBUNG DER FIGUREN

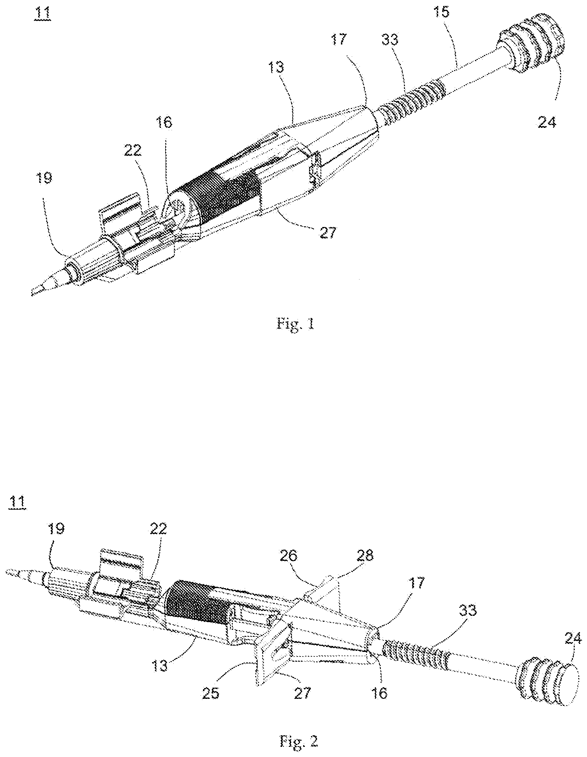

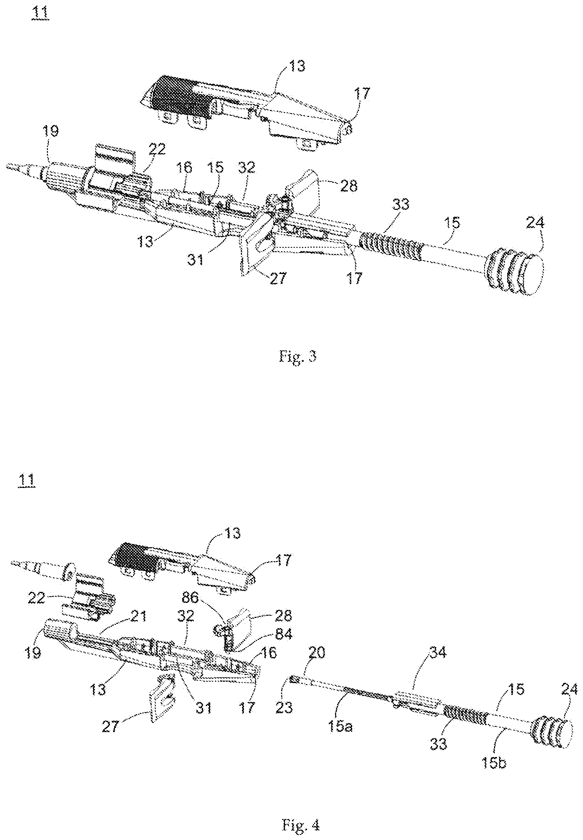

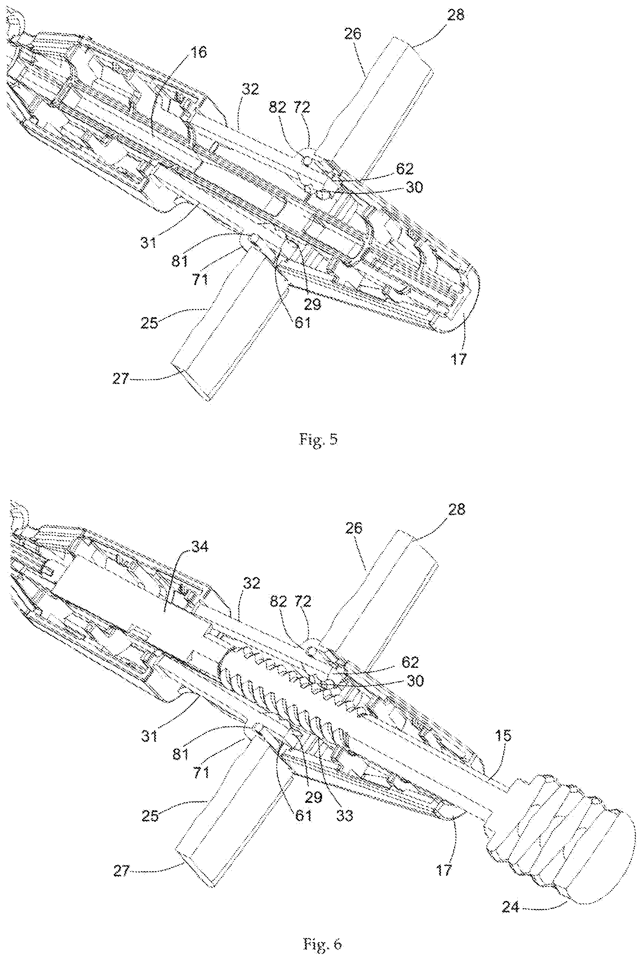

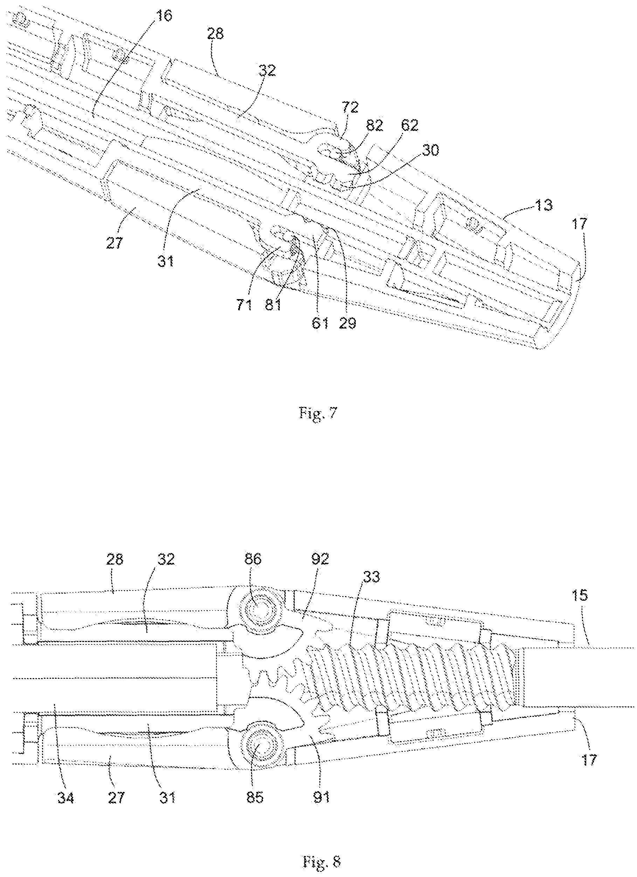

Die Fig. 1 bis 4 zeigen einen erfindungsgemässen Dualinjektor 11 für eine intraokulare Linse. In den Figuren 3 und 4 ist der Dualinjektor jeweils in unterschiedlichem Masse in seine Einzelteile zerlegt dargestellt. In den Figuren 5 bis 12 werden Details des Dualinjektors gezeigt. Der Injektor dient dem Injizieren der Linse oder einem cornealen Endotheltransplant in ein Auge. Der Dualinjektor vereinigt eine Stossfunktion und eine Schraubfunktion, sodass der Injektor in Stossfunktion als Stossinjektor (Fig. 2) oder in Schraubfunktion als Schraubinjektor (Fig. 1) verwendet werden kann, wobei jederzeit beliebig von einer Funktion in die andere und wieder zurück gewechselt werden kann. Der Injektor 11 beinhaltet zumindest ein Injektorgehäuse 13 und eine Kolbenstange 15. Das Injektorgehäuse 13 ist länglich ausgebildet mit einer ersten Öffnung an einem proximalen Ende 17, durch welche die Kolbenstange 15 eingeführt wird und einer zweiten Öffnung an einem distalen Ende 19, durch welche die Linse mittels Kolbenstange 15 ausgestossen wird. Das distale Ende 19 kann als Düse ausgebildet sein oder es kann daran eine Düse angefügt sein. Der Verbindungsgang von der ersten Öffnung zur zweiten Öffnung wird im Weiteren Kolbengang 16 (Fig. 1 oder 3) genannt. Eine Aussparung 21 im Injektorgehäuse 13 ist zweckmässigerweise zur Aufnahme einer Ladevorrichtung 22 mit einer Ladekammer für eine Linse oder ein corneales Hornhauttransplantat vorgesehen. Bei in die Aussparung 21 eingesetzter Ladevorrichtung 22 ist die Ladekammer im Kolbengang 16 positioniert, so dass beim Durchfahren der Kolbenstange 15 eine in der Ladekammer geladene Linse mittels Kolbenstange 15 aus der Ladekammer Richtung Düse ausgestossen wird. Die Kolbenstange 15 weist ein distales (vorderes) Ende 23 und ein proximales (hinteres) Ende 24 auf. Das distale Ende 23 der Kolbenstange 15 ist zweckmässigerweise mit einem Stempel 20 (auch Stössel genannt), vorzugsweise einem elastischen oder viskoelastischen Stempel, z.B. einem Stempel aus Silikon, bestückt. Der Stempel dient dem schonenden Stossen der Linse. Das proximale Ende 24 der Kolbenstange 15 bildet ein Betätigungselement, welches vorzugsweise gegenüber dem Durchmesser der proximalen Öffnung 17 des Injektorgehäuses 13 vergrössert ist. Das Betätigungselement 24 kann zum manuellen Stossen und/oder Drehen (bzw. Schrauben) der Kolbenstange 15 verwendet werden.

Die Kolbenstange 15 kann vorteilhafterweise zweiteilig ausgeführt sein, d.h. beinhaltend einen vorderen Schaftbereich 15a und einen hinteren Schaftbereich 15b, wie z.B. in Fig. 4 gezeigt. Diese beiden Schaftbereiche 15a und 15b sind vorzugsweise gegeneinander drehbar gelagert. Dabei trägt einer der beiden Schaftbereiche z.B. einen Kugelkopf und der andere Schaftbereich z.B. eine Kugelpfanne, in welcher der Kugelkopf drehbar gelagert ist. In Fig. 4 ist beispielsweise eine Kolbenstange 15 gezeigt, welche zum einen einen vorderen Schaftbereich 15a mit Kugelpfanne an seinem hinteren Ende und zum andern einen hinteren Schaftbereich 15b mit Kugelkopf an seinem vorderen Ende aufweist. Kugelkopf und Kugelpfanne sind ineinander gerastet, sodass der vordere Schaftbereich 15a mittels dem hinteren Schaftbereich 15b in Kolbenstangenlängsrichtung nach vorne gestossen und nach hinten gezogen werden kann, wobei gleichzeitig die beiden Schaftbereiche 15a und 15b perpendikular zur Kolbenstangenlängsrichtung gegeneinander verdreht werden können.

Im hinteren Schaftbereich 15b der Kolbenstange 15 ist ein Gewinde (Schraubgewinde) 33 (insbesondere als Aussengewinde) ausgebildet. Im Zusammenhang mit dem Gewinde 33 dient das Betätigungselement 24 als Drehgriff zum Einschrauben der Kolbenstange 15 in das Gehäuse 13 bzw. zum Vor- und Zurückschrauben der Kolbenstange 15 (d.h. insbesondere zum Vor- und Zurückbewegen des vorderen Schaftbereichs 15a mittels Vor- und Zurückschrauben des hinteren Schaftbereichs 15b). Dualfunktionsgruppen sind an der Längsseite am Injektorgehäuse 13 ausgebildet. Eine Dualfunktionsgruppe beinhaltet wenigstens einen Flügel 27, 28 mit Griffbereich 25, 26 zum Bedienen des Injektors in Stossfunktionsstellung und wenigstens einen Gewindebereich bzw. Gewindesteg 29, 30, welcher in der Schraubfunktionsstellung der Dualfunktionsgruppe als Innengewindesteg für das Gewinde 33 der Kolbenstange 15 wirkt. Die Dualfunktionsgruppen werden durch Ein- und Ausklappen bzw. insbesondere Ein- und Ausschwenken der Flügel 27, 28 bedient. Zum einen können die Flügel 27, 28 und somit die Griffbereiche 25, 26 von der Injektorwand weg nach aussen abgeklappt werden, wo die Griffbereiche 25, 26 als Flügelgriffe wirken. Abgeklappt sind die Flügelgriffe 27, 28 längsseitig, nach aussen abstehend am Injektorgehäuse 13 ausgebildet und dienen zusammen mit dem Betätigungselement 24 am proximalen Ende der Kolbenstange 15 als Griffe zur manuellen (ggf. einhändigen) Bedienung des Injektors 11 in Stossfunktion, insbesondere zum Vorstossen der Kolbenstange 15 in Richtung des distalen Endes 19 des Injektorgehäuses 13. Die Dualfunktionsgruppen sind derart ausgestaltet, dass bei ausgeklappten Flügeln 27, 28 (Stossfunktionsstellung) die Gewindestege 29, 30 derart gegenseitig voneinander beabstandet sind, dass sie gemeinsam das Gewinde 33 der Kolbenstange 15 im Gang 16 nicht greifen können bzw. ein Durchstossen der Kolbenstange 15 durch den Kolbengang 16 nicht behindern. In der Stossfunktionsstellung der Dualfunktionsgruppe sind die Gewindestege 29, 30 somit inaktiv. Zum andern können die Flügel 27, 28 eingeklappt werden. Beim Einklappen der Flügel werden die Gewindestege 29, 30 (von der Injektorwand weg) nach Innen in den Kolbengang 16 hinein geschoben, wo die Gewindestege 29, 30, in einem gewissen engeren Abstand zueinander gesetzt zusammen einen Innengewindeabschnitt bilden, der für den Schraubabschnitt 33 am Kolben 15 als Gegengewinde wirkt.

Flügelgriff 27, 28 und Gewindesteg 29, 30 bilden somit eine Dualfunktionsgruppe, welche zur Umstellung von der Stossfunktion in die Schraubfunktion dient, wobei Flügelgriff 27, 28 und Gewindesteg 29, 30 derart zusammenwirken, dass beim Ein- bzw. Ausklappen des Flügelgriffs 27, 28 der Gewindesteg 29, 30 derart in die Kolbenstangenpassage 16 einrückt (oder anders ausgedrückt, derart Richtung Kolbenstangenachse hinrückt), dass der Gewindesteg 29, 30 ein Innengewinde für das Schraubgewinde der Kolbenstange 15 bildet und somit die Kolbenstange 15 nur durch Drehen in Richtung Injektordüse vorgetrieben werden kann, bzw. derart von der Kolbenstangenpassage 16 abrückt (oder anders ausgedrückt, derart von der Kolbenstangenachse wegrückt), dass der Gewindesteg 29, 30 kein Innengewinde mehr bildet und somit die Kolbenstange 15 durch Stossen vorgetrieben werden kann.

Die Kolbenstange 15 ist zweckmässigerweise im Injektorgehäuse 13 (mit möglichst geringem Spiel) geführt, und zwar unabhängig von der Funktionsart Stossfunktion oder Schraubfunktion. Eine Führung kann zum Beispiel dadurch erreicht werden, dass zum einen an der Kolbenstange 15 eine Führungsstruktur 34 ausgebildet ist und zum anderen am Injektorgehäuse 13 dem Querschnitt der Führungsstruktur 34 angepasste Gegenstrukturen (z.B. eine Art Leitplanken für die Führungsstruktur 34) als Innenwandbereiche der Kolbenstangenpassage 16 ausgestaltet sind. Zweckmässigerweise kann zum Beispiel die Führungsstruktur 34 die Kugelpfanne beinhalten. In Fig. 4 ist die Führungsstruktur 34 beispielsweise am hinteren Ende des vorderen Schaftbereichs 15a als verdickter Kolbenstangenquerschnittsbereich mit Kugelpfannenaushöhlung ausgebildet. Wobei die Aushöhlung derart ausgebildet ist, das sie den Kugelkopf, welcher am vorderen Ende des hinteren Schaftbereichs 15b ausgebildet ist, aufnehmen kann. Die Führungsstruktur 34 dient somit vorteilhafterweise als Führung der Kolbenstange 15, insbesondere des vorderen Teils 15a der Kolbenstange 15, und zum anderen als Kugelpfanne für die Aufnahme des Kugelkopfes des drehbaren hinteren Teils 15b der Kolbenstange 15. Der vordere Schaftbereich 15a ist in der hier gezeigten Ausführung (insbesondere aufgrund seiner im Querschnitt nicht kreisrunden Ausführung und einer entsprechenden Kolbengangquerschnittsausgestaltung) nicht drehbar, sodass sichergestellt ist, dass eine Linse beim Vorstossen mittels der Kolbenstange 15 nicht gedreht wird.

Flügelgriff 27, 28 und dazugehöriger Gewindebereich 29, 30 sind jeweils Teil einer Funktionsgruppe, d.h. der Dualfunktionsgruppe. Diese sind insbesondere in den Fig. 5 bis 9 dargestellt. Zweckmässigerweise sind zwei Dualfunktionsgruppen an der Längsseite des Injektorgehäuses 13 vorgesehen. Eine vorteilhafte Dualfunktionsgruppe beinhaltet einen um eine Schwenkachse 35, 36 in Bezug auf das Injektorgehäuse schwenkbaren bzw. klappbaren Flügel 27, 28 mit einem Exzenter 81, 82 sowie eine in Bezug auf das Injektorgehäuse 13 auslenkbare Gabelhalterung 31, 32 beinhaltend zwei Schenkel, einen inneren Schenkel 61, 62 und einen äusseren Schenkel 71, 72. An der Gabelhalterung 31, 32 ist ein Gewindebereich 29, 30 ausgebildet. Wie in den Figuren gezeigt, kann der Gewindebereich 29, 30 schenkelaussenseitig am inneren Schenkel der Gabel ausgebildet sein (Fig. 5 bis 8). Gabelhalterung 31, 32 und Flügel 27, 28 sind separat voneinander, auslenkbar bzw. schwenkbar am Injektorgehäuse 13 ausgebildet und derart zueinander angeordnet, dass der Flügel 27, 28 mittels Exzenter 81, 82 zwischen die Schenkel der Gabelhalterung 31, 32 eingreift, sodass beim Ausklappen des Flügels 27, 28 die Gabel mittels Exzenter 81, 82 vom Kolbengang 16 weggezogen und damit der Gewindesteg 29, 30, welcher an der Gabelhalterung 31, 32 bzw. insbesondere am Gabelschenkel ausgebildet ist, aus dem Kolbengang 16 herausgezogen wird und beim Einklappen des Flügels 27, 28 die Gabel mittels Exzenter 81, 82 zum Kolbengang 16 hingeschoben und damit der Gewindesteg 29, 30 in den Kolbengang 16 hineingestossen wird.

Aus Vorversuchen der Anmelderin erging, dass auf Gewindeteile, welche am Schraubgewinde der Kolbenstange als Innengewinde eingesetzt werden, beim Schrauben recht hohe Kräfte wirken. Diese Kräfte treiben beim Schrauben die Gewindeteile von der Kolbenstange weg, sodass im Gegenzug eine recht hohe Kraft aufgebracht werden muss, um die Gewindeteile auf Position zu halten und dadurch den Schraubmodus aufrecht zu erhalten. Die in den Fig. 1-9 gezeigte erfindungsgemässe Ausführung mit Exzenter am Klappflügel zum Einstossen eines verschieblichen Gewindeteils erwies sich als besonders vorteilhaft, da die eingeklappten Flügel durch den beim Schrauben auf das Gewinde ausgeübte Druck nicht selbständig ausgeklappt werden. Somit muss der Operateur während des Schraubvorgangs nicht darauf achten, die Flügel anzudrücken, sondern kann sich auf die Vorwärtsbewegung des Kolbens und somit der zu injizierenden Linse konzentrieren. In der vorliegenden Ausführung wird dies dadurch erreicht, dass die auf das Gewinde wirkenden Kräfte, so auf die Flügeldrehachse 35, 36 übertragen werden, dass die resultierenden Kräfte im Wesentlichen keine öffnende Wirkung auf die Flügel erzeugen.

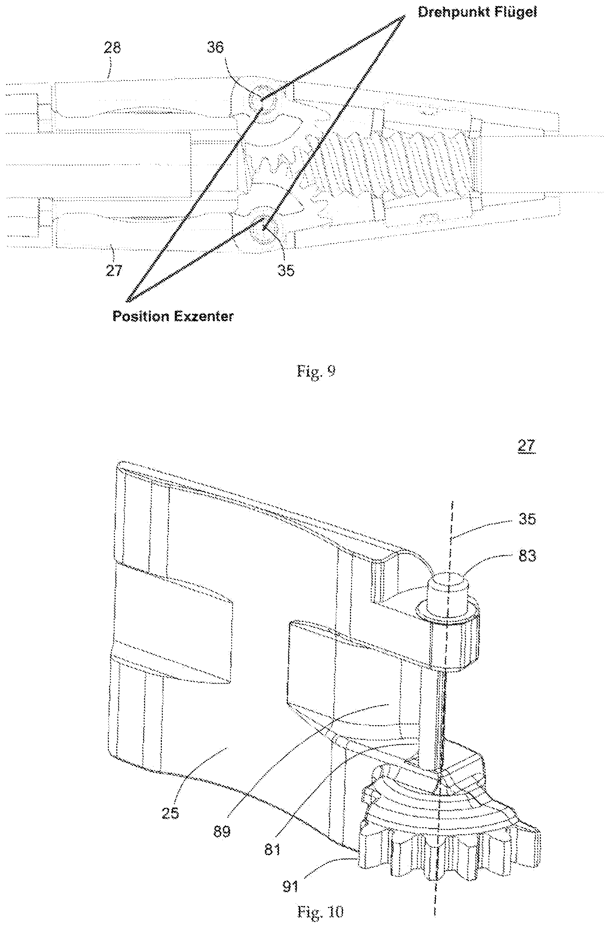

In der in Fig. 9 gezeigten Ausführung bilden zweckmässigerweise die Grifferstreckung des Flügels 27 bzw. 28 ab Drehachse 35 bzw. 36 und der von der jeweiligen Drehachse 35, 36 beabstandet angelegte Exzenter 61 bzw. 62 einen Winkel von z.B. ungefähr 90 Grad um die jeweilige Drehachse 35, 36. Insbesondere sind Winkelbereiche von 90°±5°, weiter bevorzugt von 90°±2° und noch weiter bevorzugt von 90°±1° sinnvoll. Die mittels Schraubgewinde 33 auf die Gewindestege 29, 30 wirkenden Kräfte, können dabei in eingeklappter Flügelstellung derart auf die Exzenter 61, 62 übertragen werden, dass die resultierenden Kräfte im Wesentlichen keine öffnende Wirkung auf die Flügel erzeugen sondern vielmehr die eingeklappte Stellung erhalten. Insbesondere, ist die konstruktive Ausführung derart angelegt, dass die resultierende Kraft des geschraubten Kolbens 15 auf den jeweiligen Exzenter 61, 62 in eine Richtung wirkt, welche vom Exzenter ausgehend auf die Drehachse des Flügels gerichtet ist oder bevorzugt welche vom Exzenter aus in Bezug auf die Injektionsrichtung auf oder besonders bevorzugt vor die Drehachse (bzw. in Fig. 9 vor den Drehpunkt) des jeweiligen Flügels gerichtet ist.

Die Gabelhalterung 31, 32 ist vorzugsweise geformt als ein mit einem ersten Ende am Injektorgehäuse 13 befestigter Stil und mit am freien, zweiten Ende ausgebildeter zweischenkliger Vergabelung. Aufgrund der konstruktiven Struktur und der Materialbeschaffenheit ist das vergabelte Ende der Halterung 31, 32 beweglich bzw. verschwenkbar gegenüber dem am Injektorgehäuse fixierten bzw. im Injektorgehäuse integrierten Ende. Alternative Ausführungen sind denkbar, zum Beispiel könnte das erste Ende der Gabelhalterung mittels einer Drehachse am Gehäuse befestigt sein. Die Gabelhalterung 31, 32 erstreckt sich zweckmässigerweise entlang der Längsrichtung des Injektorgehäuses und ersetzt auf einem Teilbereich die Injektorgehäusewand, was Platz für die Verschiebung der Gewindestege 29, 30 schafft.

Zur drehbaren Verankerung der Flügel im Gehäuse 13 beinhaltet jeder Flügel 27, 28 vorzugsweise zwei Achselemente 83, 84, 85, 86, welche z.B. als Stifte oder Einbuchtungen ausgebildet sein können. Im Gehäuse sind zur Aufnahme der Flügel entsprechende Gegenstrukturen zu den Achselementen 83, 84, 85, 86 ausgebildet. Die Achselemente bzw. eine Verbindungslinie zwischen den zwei Achselementen 83, 84 bzw. 85, 86 eines Flügels 27, 28 definiert die Drehachse bzw. Schwenkachse 35, 36 des Flügels 27, 28. Im Weiteren beinhaltet jeder Flügel 27, 28 einen Griffbereich 25 zum Bedienen des Injektors in Stossfunktionsstellung. Jeder Flügel 27, 28 weist einen Exzenter 81, 82 auf, mittels welchem je ein verschieblicher Gewindebereich 29, 30, in eine Stossfunktionsstellung oder in eine Schraubfunktionsstellung gebracht werden kann. In der Schraubfunktionsstellung (Fig. 1, 7 und 8) wirkt der Gewindebereich 29, 30 als Innengewindesteg für das Schraubgewinde 33 der Kolbenstange 15. In der Stossfunktionsstellung (Fig. 2, 5 und 6) ist der Gewindebereich 29, 30 möglichst aus dem Kolbengang 16 heraus Richtung Gehäusewand bzw. Richtung Drehachse des jeweiligen Dualelements 27, 28 geschoben, sodass der Gewindebereich 29, 30 ein Durchstossen der Kolbenstange 15 mit Schraubgewinde 33 durch den Kolbengang 16 nicht behindern kann. Die Position des jeweiligen Exzenters 81, 82 ist exzentrisch bezüglich der jeweiligen Flügeldrehachse 35, 36. Die Flügeldrehachse 35, 36 ist durch zwei in Achsrichtung beabstandete Achselemente 83, 84, 85, 86 definiert und derart ausgestaltet, dass dort wo die konstruktive Ausbildung der Drehachse 35, 36 unterbrochen ist (d.h. zwischen den Achselementen 83, 84, 85, 86), der Exzenter 81, 82 exzentrisch zur Flügeldrehachse 35, 36 ausgebildet ist. Der Exzenter ist vorzugsweise als Zylinderform am Flügel 27, 28 ausgestaltet, z.B. als kreisrunder Zylinder, und zweckmässigerweise parallel zur Drehachse 35, 36 des jeweiligen Flügels ausgerichtet.

Durch Umstellen der Flügel 27, 28 von einer ersten Position (ausgeklappte Flügel) in eine zweite Position (eingeklappte Flügel) kann ein Funktionswechsel von Stossfunktion auf Schraubfunktion vorgenommen werden. Bei Umstellung der der Flügel 27, 28 von einer ersten Position (ausgeklappte Flügel) in eine zweite Position (eingeklappte Flügel) werden gleichzeitig auch die Exzenter 81, 82 von einer ersten Position (mit einem ersten gegenseitigen Abstand bei ausgeklappten Flügeln) in eine zweite Position (mit einem zweiten, geringeren gegenseitigen Abstand bei eingeklappten Flügeln) gestellt.

In Stossfunktionsstellung ist das Flügelelement 27 vom Injektorgehäuse nach aussen abstehend (insbesondere vom Injektorgehäuse weggeschwenkt und gegen ein Weiterschwenken zum Betätigungselement 24 bzw. zum proximalen Gehäuseteil hin blockiert), gleichzeitig ist der Gewindebereich 29, 30 in Ruhestellung bzw. aus dem Kolbengang 16 herausgezogen und in funktionsloser Stellung (d.h. ausser Funktion). In Schraubfunktionsstellung wird der Gewindebereich 29, 30 nach innen in den Kolbengang 16 gedrückt, gleichzeitig ist der Flügel 27, 28 eingeklappt und bildet vorzugsweise ein Teil der Injektorgehäusewand aus bzw. ist in die Injektorgehäusewand eingesenkt. Die Umstellung von einer Funktionsstellung in die andere kann zweckmässigerweise durch Drehen oder Klappen um die Drehachse 35, 36 erfolgen.

Durch eine Verzahnung (91, 92) kann die Bewegung der beiden Flügel 27, 28 und somit der beiden Dualfunktionsgruppen synchronisiert werden, so dass nur ein Flügel 27 oder 28 bzw. ein- oder ausgeklappt wird und der zweite sich dazu symmetrisch verhält. Hierfür weist jeder der beiden Flügel 27, 28 ein zu seiner Drehachse 35, 36 konzentrisches Zahnrad oder zumindest Teilzahnrad 91, 92 auf. Die beiden Zahnräder oder Teilzahnräder sind insbesondere derart ausgestaltet und am Gehäuse 13 installiert derart miteinander verzahnt, dass sich beide Flügel nur synchron aus- und einklappen lassen.

In der Stossfunktion dienen die ausgeklappten Flügel 27, 28 zusammen mit dem Betätigungselement 24 der Kolbenstange als Griffe zur manuellen Bedienung des Injektors zum (im Wesentlichen geraden) Vorstossen der Kolbenstange 15 in Richtung des distalen Endes 19 des Injektorgehäuses 13. Die Gewindestege 29, 30 sind bei ausgeklappten Flügeln 27, 28 mit den Exzentern 81, 82 in einer ersten Position derart voneinander beabstandet, dass die Kolbenstange 15 ohne Behinderung durch die Gewindestege 29, 30 durch den Kolbengang 16 gestossen werden kann. Bedient wird der Dualinjektor in Stossfunktionsstellung zum Beispiel, indem gleichzeitig einerseits von hinten auf das Betätigungselement 24 und andererseits in entgegengesetzter Kraftrichtung auf die Flügelgriffbereiche 27, 28 gedrückt wird - dies vorzugsweise einhändig (insb. z.B. per einhändigem Dreifingergriff).

In der Schraubfunktion dienen die aufgrund einer zweiten Exzenterposition nach innen, d.h. in den Kolbengang hinein, gesetzten Gewindestege 29, 30 als Innengewinde für das Kolbenstangengewinde 33, sodass die Kolbenstange 15 durch eine manuell drehende Betätigung des Betätigungselements 24 eingeschraubt werden kann und dadurch ein Vorstossen der Kolbenstange 15 in Richtung des distalen Endes 19 des Injektorgehäuses 13 unter Drehung um die eigene Achse (Schraubbewegung) bewirkt wird. Bedient wird der Dualinjektor in Schraubfunktionsstellung zum Beispiel, indem gleichzeitig einerseits mit einer Hand das Betätigungselement 24 gedreht wird und andererseits mit der anderen Hand das Injektorgehäuse 13 festgehalten wird (d.h. z.B. durch zweihändige Bedienung).

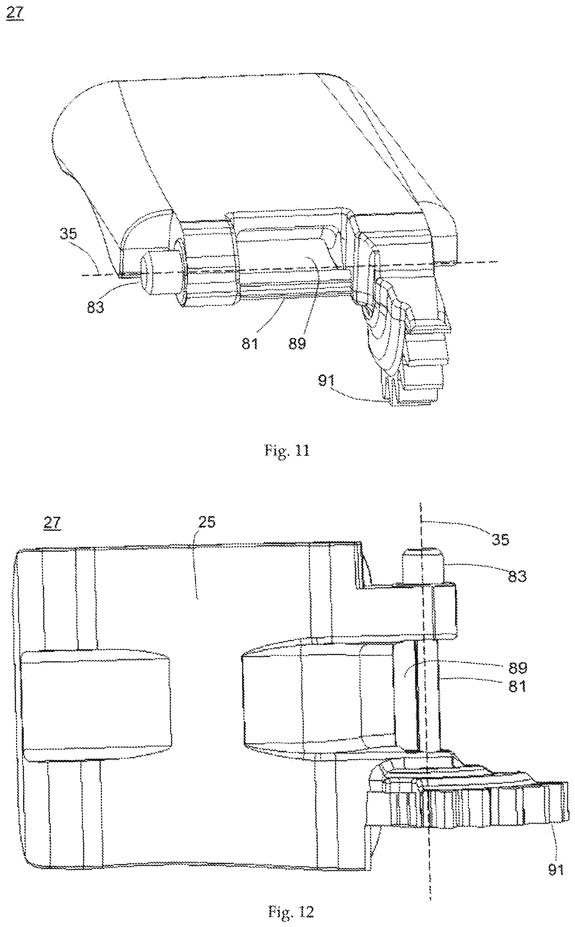

In Fig. 10, 11 und 12 ist ein exemplarischer Flügel 27, wie er zum Beispiel in der Ausführung nach Fig. 1 bis 8 eingesetzt sein kann, gezeigt. In den Fig. 10 und 11 ist insbesondere der Versatz zwischen dem Exzenter 81 und der Drehachse 35 (Drehachse als strichlierte Linie angezeigt), welche durch die Achsverlängerung des Achselements 83 definiert ist, ersichtlich. Dieser Versatz entspricht dem in Fig. 9 angezeigten Versatz zwischen dem Drehpunkt 35, 36 des jeweiligen Flügels 27, 28 und der entsprechenden Exzenterposition. Dadurch, dass der Exzenter 81 parallel zur Drehachse 35 des Flügels 27, jedoch zwischen den voneinander beabstandeten Elementen 83, 85, welche die Drehachse 35 des Flügels 27 definieren, ausgebildet ist, kann der Exzenter von den Gabelschenkeln 61, 71 derart eingefasst werden, dass beim Umklappen des Flügels 27 die Gabelschenkel 61, 71 durch die Exzenterbewegung mit dem Exzenter 81 zusammenwirkend versetzt werden. Damit die Halterung 31, 32 bzw. deren vergabelte Schenkel 61, 62, 71, 72 den Exzenter 83, 85 umfassen kann bzw. können, ist am Flügel 27, 28 eine Aussparung 89 vorgesehen. Während in den Fig. 2 bis 4 der Flügel 27 im Gegensatz zu Flügel 28 mit einer optional vergrösserten Aussparung dargestellt wurde, wird in den Fig. 10 bis 12 auf eine derartige Vergrösserung der Aussparung verzichtet.

Damit der Injektor 11 möglichst kostengünstig ist, wird er vorzugsweise im Wesentlichen aus einem geeigneten Kunststoff hergestellt, z.B. per Spritzguss. Insbesondere werden das Gehäuse 13 inklusive Gabelhalterung 31, 32, bzw. mehrere Gehäuseteile, die Kolbenstange 15, die Flügel 27, 28, und die Ladekammer 22 vorzugsweise aus einem Kunststoff, z.B. ABS, Polycarbonat und/ oder Polyporopoylene, erzeugt.

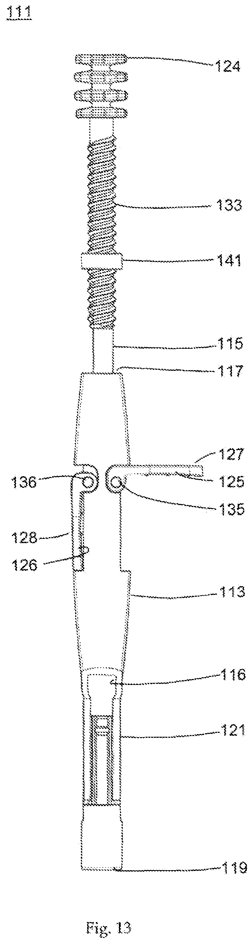

In Fig. 13 bis Fig. 17 ist eine schematische Ausführung eines Dualinjektors 111 in Kombination mit einem Anschlagelement 141 gezeigt. Hierbei sind Stoss- und Schraubstellung der Flügel in ein und derselben Figur einander gegenübergestellt gezeigt. Der linke Flügel 128 liegt mit seinem Griffbereich 126 am Injektorhäuse 113 längsseitig an und ist somit in Schraubfunktionsstellung gezeigt. der rechte Flügel 127 steht im Wesentlichen rechtwinklig vom Injektorhäuse 113 ab und ist somit in Stossfunktionsstellung gezeigt. Diese Darstellungsart dient lediglich dazu, zu verdeutlichen, dass beide Funktionen verwendet werden können bzw. zwischen diesen gewählt oder gewechselt werden kann. Bezüglich Details zur Funktionalität in Stoss- und Schraubstellung wird zudem auf die Fig. 1-13 verwiesen.

Zur Benutzung des Dualinjektors 111 als Stossinjektor sollten beide Flügelgriffe 127, 128 ausgeklappt sein. Durch Druck auf das Betätigungselement 124 (z.B. per Daumen oder Handfläche) und durch gleichzeitigen Gegendruck auf die Griffflächen 125 der beiden Flügel 127, 128 kann die Kolbenstange 115 bzw. deren Spitze 123 zum distalen Ende 119 des Injektorgehäuses 113 (und somit zu einer dort integrierten oder montierten Injektordüse) hin gestossen werden.

Zur Benutzung des Dualinjektors 111 als Schraubinjektor sollten beide Flügelgriffe 127, 128 eingeklappt sein. In dieser Schraubfunktionsstellung der Dualelemente 127, 128 kann die Kolbenstange 115 durch Drehen des Betätigungselements 124 um die Längsachse der Kolbenstange 115 in den Kolbengang 116 hinein getrieben werden kann.

Der Flügelgriff 127 weist eine Grifffläche 125 auf, welche vorteilhafterweise mit Rippen gegen ein Abrutschen bzw. für einen guten Halt ausgestattet ist. Die Grifffläche 125 ist in Stossfunktionsstellung des Flügels 127 vorzugsweise im Wesentlichen perpendikulär zur Kolbenstangenstossrichtung ausgerichtet und die Grifffläche 125 weist zur distalen Kolbenstangenspitze hin. In Schraubfunktionsstellung hingegen ist der Flügel 128 an das Injektorgehäuse 113 angeschmiegt und bildet vorzugsweise zusammen mit der Aussenseite des Gehäuses 113 im Wesentlichen eine kontinuierlichen Griffelstiel.

Das Anschlagelement 141 (wie in Fig. 13 bis 17 angezeigt), welches die Kolbenstange 115 umfasst bzw. auf diese aufgezogen ist, ist vorzugsweise aus einem deformierbaren und/oder komprimierbaren Material gefertigt, z.B. aus Silikonmaterial oder TPE (thermoplastisches Elastomer), vorzugsweise aus einer 1 mm bis 6 mm, bevorzugt 2 mm bis 5 mm, weiter bevorzugt 3 mm bis 4 mm dicken Scheibe eines dieser Materialien. Die Shore-Härte des Silikonmaterials ist vorzugsweise mindestens 20 Shore und höchstens 80 Shore, vorzugsweise 30 Shore bis 70 Shore, idealerweise 40 Shore bis 60 Shore. Das Anschlagelement 141 ist vorzugsweise ringartig, d.h. als Ring (Ringscheibe), insbesondere als geschlossener Ring (geschlossene Ringscheibe) ausgeführt.

Im Folgenden wird insbesondere auf die vorteilhafte Verwendung eines Anschlagelements 141 in Kombination mit einem Stossinjektor, einem Schraubinjektor oder einem Dualinjektor 111 eingegangen.

In Fig. 13 ist das Anschlagelement 141 verschiebbar über den hinteren Bereich des Schafts der Kolbenstange aufgezogen (im Vergleich Fig. 1-4). In einer Ausgangsstellung (Fig. 13) kann das Anschlagelement 141, z.B. verschiebbar ungefähr mittig über den hinteren Bereich des Schafts der Kolbenstange 115 aufgezogen sein. Insbesondere kann dabei das Anschlagelement 141 in einem gewissen Abstand dem Betätigungselement 124 vorgelagert sein, wo es vorzugsweise in einer Kerbe (nicht gezeigt) und/oder auf dem Gewinde 133 sitzend eine bestimmte Vorschubposition markiert, an welcher vor dem weiteren Vorbewegen der Kolbenstange 115 eingehalten werden soll, z.B. sobald das Anschlagelement an den Injektorkörper stösst. Dies ist besonders im Fall von vorgeladenen Injektorsystemen (d.h. bei Systemen, bei welchen die Linse schon bei Lieferung in den Injektor geladen ist) nützlich. Hierbei dient das Anschlagelement 141 zum einen der Einstellung und Einhaltung einer gewissen Vorschubposition des distalen Endes 123 der Kolbenstange 115, an welcher angehalten und gewisse Vorbereitungsschritte bei noch geöffneter Ladekammer durchgeführt werden können. Diese Vorbereitungsschritte können beispielsweise das Anlegen der Haptiken der Linse an die Optik mittels des Silikonstempels auf der Kolbenstange oder auch das Entfernen des Linsenhalters bei vorgeschobener Kolbenstange beinhalten.

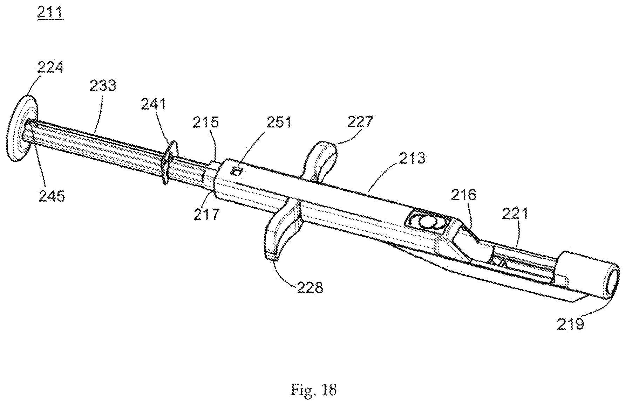

In Fig. 13 ist die Kolbenstange 115 auf eine erste Vorschubposition in das Injektorgehäuse 113 eingeführt. Diese Vorschubposition kann durch einen Federclip gesichert sein (das Konzept der Federclipsicherung ist in Fig. 13-17 nicht gezeigt, jedoch in Fig. 18 angedeutet anhand Bezugszeichen 251, welches eine Federcliparretierung zeigt), sodass diese erste Position beim Einschieben leichter gefunden, eingestellt und gegebenenfalls gehalten werden kann. Ein Weiterschieben nach Überbrücken der Federkraft ist vorgesehen. Der Federclip verhindert, dass der Kolben aus dem Injektorgehäuse rückseitig herausfallen kann, da die Kolbenstange zwar vorwärts, jedoch nicht mehr rückwärts aus der ersten Vorschubposition herausbewegt werden kann. Das Anschlagelement 141 ist auf dem hinteren Schaftteil der Kolbenstange 115, welcher aus dem Gehäuse 13 deutlich sichtbar vorsteht, verschiebbar angebracht. Ein verschiebbares Anschlagelement 141 wird hierin im Weiteren auch Schlitten genannt. Das verschiebbare Anschlagelement 141 ist an seinem äusseren Durchmesser vorzugsweise grösser ausgebildet als die Öffnung am proximalen Ende 117 des Injektorgehäuses 113. Dadurch wird gewährleistet, dass das Anschlagelement 141 beim Einschieben der Kolbenstange 115 in das Injektorgehäusee 113 nach dem Auflaufen auf das proximale Ende 117 des Injektorgehäuses 113 auf der Kolbenstange 115 Richtung Betätigungselement 124 nach hinten geschoben werden kann.

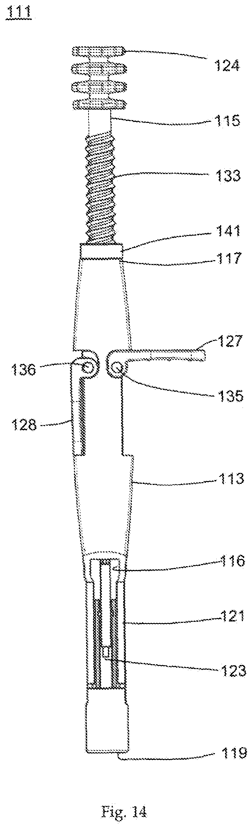

In Fig. 14 ist die zweite Vorschubposition der Kolbenstange 115 gezeigt. Hier stösst das Anschlagelement 141 erstmalig an das Injektorgehäuse an 113. Jedoch hat das Anschlagelement 141 mit Bezug auf die Kolbenstange 115 noch dieselbe Position inne wie in Fig. 13. In bzw. aus dieser Kolbenposition können Vorbereitungsschritte bei noch geöffneter Ladekammer durchgeführt werden. Diese Vorbereitungsschritte können beispielsweise das Anlegen der Haptiken der Linse an die Optik mittels des Silikonstempels auf der Kolbenstange oder auch das Entfernen des Linsenhalters bei vorgeschobener Kolbenstange beinhalten. Weitere Vorbereitungsschritte sind denkbar, beinhalten jedoch stets das Vorschieben der Kolbenstange bis das Anschlagelement spürbar am Injektorgehäuse anschlägt. Erst dann wird die Ladekammer geschlossen und die Kolbenstange weiter vorgefahren.

Das Anschlagelement 141 liegt optional in einer Kolbennut oder in dem Gewinde (nicht sichtbar da an der Kolbenstange unter dem aufgesetzten Anschlagelement liegend). Vorzugsweise benötigt es deshalb eine gewisse Überwindungskraft (z.B. 3-4 Newton), um die Kolbenstange 115 aus der zweiten Vorschubposition weiter in das Injektrogehäuse 113 einzuschieben. Diese Überwindungskraft wird zweckmässigerweise als Anschlag bzw. erhöhter Widerstand vom Anwender gefühlt, sodass zum einen das Einschieben genau an dieser zweiten Vorschubposition spürbar und sichtbar gestoppt werden kann (und ein Überfahren der zweiten Vorschubposition so verhindert wird) und zum andern durch Überwinden des Widerstands die Kolbenstange 115 kontrolliert und mit nur geringem Kraftaufwand weiter in das Injektorgehäuse geschoben werden kann. Nach dem Überwinden der zweiten Vorschubposition rutscht das Anschlagelement 141 beim weiteren Stossen oder Drehen der Kolbenstange 115 in den Kolbengang 116 auf der Kolbenstange 115 bis zum bzw. auf Anschlag am Betätigungselement 124 nach hinten.

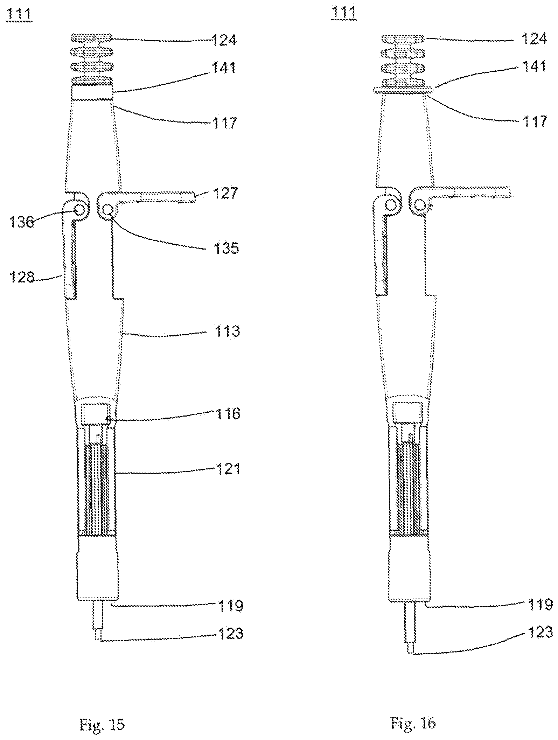

In Fig. 15 ist die Kolbenstange 115 bis auf eine dritte Vorschubposition in das Injektionsgehäuse 113 eingeführt. In dieser Position liegt das Anschlagelement 141 zum einen weiterhin am proximale Ende 117 des Injektorgehäuses 113 an und zum andern liegt das Anschlagelement 141 gleichzeitig am Betätigungselement 124 an. In dieser dritten Vorstossposition sollte idealerweise die Linse ganz ausgestossen sein.

Für den Fall, dass die Linse aus irgendwelchen Gründen (z.B. bewusst kurze Wahl der Kolbenstange zur Vermeidung des Austretens des Silikonstempels, erhöhte Reibung durch Verwendung hochvisköser viskoelastischer Flüssigkeit, niedrige Temperaturen, langsame und stockende Injektionsweise, u.s.w.) noch nicht ganz ausgestossen ist, kann das Anschlagelement 141 durch ein weiteres Vorstossen um zweckmässigerweise maximal ein paar wenige Millimeter unter sich erhöhendem manuellem Kraftaufwand zusammengestaucht bzw. komprimiert werden (insoweit es aus einem deformierbaren bzw. komprimierbaren Material, z.B. aus Silikon, gefertigt ist) bis die Linse ganz ausgestossen ist. Das aufgrund manuellen Drucks komprimierte bzw. gestauchte Anschlagelement 141 ist in Fig. 16 dargestellt. Dabei ist die Kolbenstange 115 bis auf eine vierte Vorschubposition eingestossen. Durch Komprimieren des Anschlagelements 141 kann mit der Kolbenstangenspitze 123 ein Reservevorschubbereich erreicht werden, um eine Linse, welche bei der dritten Vorschubposition noch nicht vollständig ausgestossen ist, gegebenenfalls vollständig aus dem Injektor ausstossen zu können. Durch die zur Stauchung des Anschlagelements nötige zusätzliche Kraftanstrengung, wird der Operateur taktil auf den unmittelbar bevorstehende Ausstoss der Linse vorbereitet und ein ungewollt zu weites Durchstossen der Kolbenspitze bzw. eines darauf sitzenden Stempels verhindert.

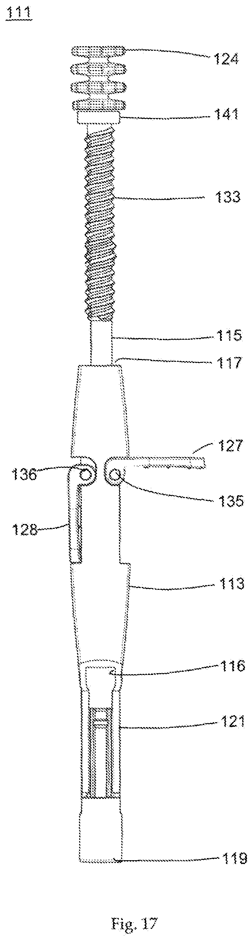

In einer alternativen Ausgangsstellung, wie in Fig. 17 gezeigt, kann das Anschlagelement 141 von Beginn an an das Betätigungselement 124 angrenzend angeordnet sein. In diesem Fall dient das Anschlagelement 141 vornehmlich zur Einstellung und Kontrolle der genauen Endposition des distalen Endes 123 der Kolbenstange 115, sodass der Anwender kurz vor Ausstoss der Linse zunächst einen klaren Anschlag der Kolbenstange bzw. des Betätigungselements 124 an das Gehäuse 117 verspürt und ein auf der Kolbenstangenspitze 123 sitzender Stempel nicht unnötig weit aus der Injektionsdüse herausgestossen wird. Bei Bedarf (also bei noch nicht vollständig ausgetretener Linse) ist aber ein weiteres Vorschieben des Kolbens 115 von zweckmässigerweise einigen Millimetern unter zusätzlicher Kraft möglich, indem dabei das Anschlagelement 141 gestaucht wird. Auf diese Art entsteht ein flexibler Reservebereich für die Vorschublänge der Kolbenstange 115 bzw. deren Spitze 123 (ohne dass der klare erste spürbare Anschlag verloren geht). Diese alternative Ausgangsstellung (gemäss Fig. 17) wird vorteilhafterweise bei Systemen angewendet, bei denen keine weiteren Vorbereitungsschritte während des Injektionsprozesses nötig sind.

In einer alternativen Ausführung gemäss Fig. 18, wird ein Stossinjektor 211 mit verschieblichem und im Wesentlichen nicht komprimierbarem Anschlagelement 241 (auch Schlitten genannt) vorgestellt. Nicht komprimierbar bedeutet hierbei, dass das Anschlagelement 241 zumindest bei Beanspruchung gemäss normaler Benutzung des Stossinjektors 211 im Wesentlichen nicht komprimierbar ist. Der Stossinjektor weist vorzugsweise am Injektorgehäuse 213 Flügelgriffe 227, 228 und am proximalen Ende der Kolbenstange 215 ein Betätigungselement 224 auf. Die Kolbenstange 215 weist im hinteren Bereich des Kolbenstangenschafts 233 eine Rampe 245 auf, welche vom Kolbenstangenshaft her zum Kolbenstangenende bzw. zum Betätigungselement 224 ansteigend ausgeführt ist. Das Anschlagelement 241 sitzt vorzugsweise in einer Kolbennut (nicht sichtbar) der Kolbenstange 215.

Von der in Fig. 18 gezeigten Vorschubstellung (ähnlich der mit Bezug auf Fig. 13 gezeigten ersten Vorschubstellung) aus kann die Kolbenstange 215 in zwei Etappen und optional einer Reserveetappe in das Injektorgehäuse 213 eingeschoben werden.

Von der in Fig. 18 gezeigten ersten Vorschubstellung wird die Kolbenstange 215 weiter in das Injektorgehäuse 213 geschoben bis der Schlitten 241 am proximalen Ende 217 des Injektorgehäuses 213 anliegt (insb. ohne dass dabei der Schlitten 241 in Achsrichtung gegenüber dem Schaft der Kolbenstange 215 verschoben wurde). Diese Position oder Stellung wird zweite Vorschubposition oder -stellung genannt (ähnlich der mit Bezug auf Fig. 14 gezeigten zweiten Vorschubstellung). Dabei sitzt der Schlitten noch in der Kolbennut der Kolbenstange 215, sofern eine Kolbennut am Schaft vorgesehen ist.

Wird die Kolbenstange 215 weiter in das Injektorgehäuse 213 gestossen, wird gleichzeitig der Schlitten 241 dem Schaft 233 der Kolbenstange 215 entlang nach hinten gestossen und gegebenenfalls auf die Rampe 245 geschoben. Der Schlitten 241 lässt sich dabei weitestgehend wiederstandslos schieben. Sobald die Rampe 245 am proximalen Ende 217 des Injektorgehäuses 213 anstösst, ist die Kolbenstange 215 bis auf eine dritte Vorschubposition (ähnlich der mit Bezug auf Fig. 15 gezeigten dritten Vorschubstellung) in das Injektionsgehäuse 213 eingeführt. In dieser dritten Vorstossposition sollte indealerweise die Linse ganz ausgestossen sein.

Ist die Linse in der dritten Vorstossposition jedoch nicht ganz ausgestossen, kann durch weiteren Druck die Rampe 245 in den Rand des Injektorgehäuses 213 an seinem proximalen Ende 217 gedrückt werden. Die dabei erreichte Endposition der Kolbenstange 215 kann Reserveposition genannt werden. Die Rampe 245 kann nur unter erhöhter manueller Kraftaufwendung des Operateurs in das proximale Ende 217 des Injektorgehäuses 213 eingestossen werden.

Damit das Anschlagelement 241 über die Rampe 245 im Wesentlichen reibungslos hinweggleiten kann, ist das Anschlagelement 241 vorzugsweise entsprechend flexibel ausgestaltet, was durch eine geeignete Materialwahl und/oder konstruktiv bedingte Formgebung von Rampe 245 und Anschlagelement 241 erreicht werden kann. Zum Beispiel kann das Anschlagelement (bzw. der Schlitten) 241 als U-förmiges Klammerelement aus vorzugsweise im Wesentlichen (unter den vorgesehenen Anwendungsbedingungen) nicht komprimierbarem Material gefertigt sein, welches sich beim Herausschieben aus der Kolbenkerbe plastisch spreizt und sich dadurch dem Kolben entlang verschieben lässt und auf die Rampe 245 aufstossen lässt bis das Anschlagelement 241 effektiv am Betätigungselement 224 anstösst.

Im Weiteren folgen Anwendungsbeispiele zur Verwendung von Injektoren mit einem Anschlagelement.

### ANWENDUNGSBEISPEIL 1

Es wird ein Dualinjektor 111 wie oben beschrieben (z.B. Fig. 13-16) mit Injektorgehäuse 113 und Kolbenstange 115 bereitgestellt. Auf der Kolbenstange 115 ist ein verschiebbar angebrachtes Anschlagelement 141 aus einem Silikonmaterial vorgesehen. Das Anschlagelement ist z.B. als ringförmiges Element aus einem kompressiblen Material ausgeführt. Eine Ladevorrichtung (in den Figuren nicht gezeigt) zur Aufnahme einer Linse oder mit einer bereits vorgeladenen Linse ist in der Aussparung eingelegt. Die Kolbenstange 113 wird in die erste Vorschubposition gebracht oder ist vorzugsweise schon in dieser. Die erste Vorschubposition zeichnet sich dadurch aus, dass die Kolbenstange etwas in die proximale Öffnung 117 des Injektorgehäuses 113 eingeführt ist. Aus dieser ersten Position startend soll die Kolbenstange 115 bei weiterem Vorschieben (durch Stossen oder Schrauben) mit ihrem Vorderende 123 (welches vorzugsweise einen elastischen oder viscoelastischen Stempel aufweist) an die Linse geführt und die Linse bei weiterem Vorschieben der Kolbenstange 115 vor der Kolbenstange 115 her gestossen werden (vorzugsweise durch eine Düse hindurch) bis die Linse aus dem distalen Ende 119 des Injektorgehäuses 113 bzw. der am distalen Ende 119 ausgebildeten oder angebrachten Düse ausgestossen ist. Die erste Vorschubposition kann durch Einklicken in einen Federclip (in den Figuren 13-16 nicht gezeigt) gesichert sein. Das verschiebbare Anschlagelement 141 wird beim Einschieben der Kolbenstange 115 in den Kolbengang 116 aus der ersten Vorschubposition zusammen mit der Kolbenstange, sozusagen Huckepack auf der Kolbenstange 115, in Richtung zur proximalen Öffnung 117 des Injektorgehäuses 113 hin geschoben bis das Anschlagelement 141 am Rand der proximalen Öffnung 117 des Injektorgehäuses anschlägt und ein weiteres Einschieben der Kolbenstange 115 nur möglich ist, wenn die Kolbenstage 115 durch das Anschlagelement 141 hindurch geschoben wird bzw. das Anschlagelement 141 am Rand der proximalen Öffnung des Injektorgehäuses aufgehalten und auf der Kolbenstange entlang nach hinten rutscht. Die Position der Kolbenstange 115 beim ersten Anschlag des Anschlagelements 141 an den Rand der proximalen Öffnung 117 des Injektorgehäuses wird zweite Vorschubposition genannt. Diese Position ist visuell gut erkennbar und kann je nach Stärke einer Klemmwirkung des Anschlagelements 141 auf dem Gewinde der Kolbenstange oder bei vorhandener Quernut auf der Kolbenstange 115 auch taktil wahrgenommen werden, dadurch dass ein weiteres Vorschieben aufgrund eines höheren Widerstands (aufgrund der Reibung des Anschlagelements auf der Kolbenstange oder zumindest vorübergehend bei Überwinden der Quernut) spürbar ist.

Die zweite Vorschubposition ermöglicht die Ausführung weiterer Vorbereitungsschritte durch den Anwender noch vor dem Schliessen der Ladekammer. Ein typischer Vorbereitungsschritt könnte beispielsweise das mechanische Anlegen der Linsenhaptiken an die Linsenoptik mittels des Silikonstempels auf der Kolbenstange 115 sein. Auch macht es bei vorgeladenen hydrophilen Linsen Sinn, die Kolbenstange 115 noch vor Entfernen des Linsenhalters (eine Komponente, welche vorgeladene Linsen in der Ladekammer während Transport und Aufbewahrung arritiert) soweit vorzuschieben, dass die Linse nach Entfernen des Linsenhalters nicht mehr in proximaler Richtung aus der Ladekammer herausrutschen kann. Beide Vorbereitungsschritte erfolgen automatisch durch das Vorfahren der Kolbenstange bei noch geöffneter Ladekammer. Weitere Vorbereitungsschritte sind denkbar, erfolgen jedoch stets dadurch, dass die Kolbenstange 115 soweit vorgeschoben wird, bis das Anschlagelement 141 spürbar an der proximalen Öffnung 117 des Injektorgehäuses 113 anschlägt. Erst nach Ausführung des Vorbereitungsschrittes wird die Ladekammer geschlossen.

Um die Kolbenstange 115 aus der zweiten Vorschubposition weiter in den Gang des Injektorgehäuses 115 zu schieben wird die Kolbenstange 115 durch das verschiebbare Anschlagelement 141, welches am Rand der proximalen Öffnung 117 des Injektorgehäuses arretiert ist, hindurch eingeschoben oder eingedreht. Sobald das Anschlagelement 141 an das Betätigungselements 124 anstösst (d.h. zwischen Betätigungselement 124 und Rand der proximalen Öffnung 117 des Injektorgehäuses eingeklemmt ist), ist die dritte Vorstossposition der Kolbenstange 115 erreicht. Wenn die Linse in dieser dritten Vorstossposition noch nicht ganz ausgestossen ist, dann kann - insofern das Anschlagelement 141 aus einem kompressiblen Material besteht - durch zusätzlichen Druck auf das Betätigungselement 124 das Anschlagelement 141 zusammengestaucht werden, wodurch die Kolbenstange 115 noch etwas weiter (z.B. bis zu ein paar Millimeter) vorrückt und die Linse ausstösst.

Es wurde hier die Anwendung eines verschiebbaren Anschlagelements (vergleichend am Beispiel eines inkompressiblen Schlittens und am Beispiel eines kompressiblen Schlittens) beschrieben.

### ANWENDUNGSBEISPEIL 2

Im Unterschied zum vorhergehenden Anwendungsbeispiel 1 ist der Silikonring von Anfang an auf den hinteren Teil der Kolbenstange aufgestülpt und bis an das Betätigungselement herangeschoben (z.B. Fig. 17) (der Silikonring muss in diesem Beispiel nicht unbedingt verschiebbar auf der Kolbenstange lagern). Diese Anordnung bewährt sich vorzugsweise bei Systemen, bei welchen kein weiterer Vorbereitungsschritt notwendig ist. Beim Vorstossen der Kolbenstange wird die zweite Vorstossposition (ähnlich der vorgenannten zweiten Vorstossposition in Anwendungsbeispiel 1) taktil nicht gespürt und somit überfahren, da der Silikonring schon hinten am Betätigungselement ist. Gegebenenfalls könnte die zweite Vorstossposition aufgrund von visuellen Markern eingestellt werden. Sobald der Silikonring zwischen Betätigungselement und proximalem Ende des Injektorgehäuses klemmt, ist die dritte Vorstossposition der Kolbenstange erreicht. Ist die Linse in dieser dritten Vorstossposition noch nicht ganz ausgestossen, kann durch zusätzlichen Druck auf das Betätigungselement 124 der Silikonring 141 komprimiert werden, wodurch die Kolbenstange noch ein paar wenige Millimeter vorrückt und die Linse ausstösst.

Es wurde hier die Anwendung eines kompressiblen Anschlagelements (am Beispiel eines Silikonrings) beschrieben.

### VERGLEICH DER ANWENDUNGSBEISPIELE 1 UND 2

In den beiden Beispielen, Anwendungsbeispiel 1 und Anwendungsbeispiel 2 ist eine Vorschubreserve dadurch realisiert, dass nur durch deutlich kräftigeres Drücken oder Schrauben ein zusätzlicher Vorschub durch Komprimieren des Anschlagelements 141 erreicht wird. Das Material des Anschlagelement 141 ist dazu unter manuell aufgebrachter Kraft komprimierbar. Diese Anschlagsart kann auch als Soft-Anschlag bezeichnet werden.

Um einen nützlichen Extra-Kolbenweg zu erreichen ist die Silikonringdicke zweckmässigerweise im Bereich von mindestens 1 mm bis höchstens 6 mm, vorzugsweise im Bereich von 2 mm bis 5 mm, idealerweise im Bereich von 3 mm bis 4 mm. Dabei ist die Shore-Härte bevorzugt im Bereich von mindestens 20 Shore und höchstens 80 Shore, vorzugsweise im Bereich von 30 Shore bis 70 Shore, idealerweise im Bereich von 40 Shore bis 60 Shore.

Im ersten Ausführungsbeispiel sind die vorteilhaften Eigenschaften zweier unterschiedlicher Anschlagelemente kombiniert:
a) ein verschiebbares Anschlagelement (Schlitten), und
b) ein komprimierbares Anschlagelement (Soft-Anschlag).

Die Kombination der beiden Anschlagsmerkmale ist besonders vorteilig für die Anwendung bei einem Schraub-Injektor oder bei einem Dualinjektor, welcher als Schraubinjektor oder Druckinjektor einsetzbar ist.

### ANWENDUNGSBEISPEIL 3

Es wird ein Stossinjektor 211 wie z.B. oben in Fig. 18 beschrieben mit Injektorgehäuse 213 und Kolbenstange 215 bereitgestellt. Die Kolbenstange 215 besitzt eine zum Betätigungselement hin ansteigender Rampenkeil 245. Auf der Kolbenstange 215 ist ein verschiebbar angebrachtes Anschlagelement (auch Schlitten genannt) 241 vorgesehen, welches im Wesentlichen inkompressibel ist.

Das Anschlagelement kann z.B. als U-förmiges Klammerelement ausgeführt sein (wie in Fig. 18 dargestellt).

Eine Ladevorrichtung (nicht gezeigt) zur Aufnahme einer Linse oder mit einer bereits vorgeladenen Linse ist in der Aussparung eingelegt bzw. kann darin eingelegt werden. Die Kolbenstange 213 wird in die erste Vorschubposition gebracht oder ist schon in dieser. Die erste Vorschubposition zeichnet sich dadurch aus, dass die Kolbenstange etwas in die proximale Öffnung 217 des Injektorgehäuses 213 eingeführt ist. Aus dieser ersten Position startend soll die Kolbenstange 215 bei weiterem Vorschieben durch Stossen mit ihrem Vorderende 223 an die Linse geführt und die Linse bei weiterem Vorschieben der Kolbenstange 215 vor der Kolbenstange 215 her gestossen werden (vorzugsweise durch eine Düse (in den Figuren nicht gezeigt) hindurch) bis die Linse aus dem distalen Ende 219 des Injektorgehäuses 213 bzw. der am distalen Ende 219 ausgebildeten oder angebrachten Düse ausgestossen ist. Die erste Vorschubposition kann mittels eines Federclips gesichert sein. Das verschiebbare Anschlagelement 241 wird beim Einschieben der Kolbenstange 215 in den Kolbengang 216 aus der ersten Vorschubposition zusammen mit der Kolbenstange, sozusagen Huckepack auf der Kolbenstange 215, in Richtung zur proximalen Öffnung 217 des Injektorgehäuses 213 hin geschoben bis das Anschlagelement 241 am Rand der proximalen Öffnung 217 des Injektorgehäuses anschlägt und ein weiteres Einschieben der Kolbenstange 215 nur möglich ist, wenn die Kolbenstage 215 durch das Anschlagelement 241 hindurch geschoben wird bzw. das Anschlagelement 241 am Rand der proximalen Öffnung des Injektorgehäuses aufgehalten der Kolbenstange entlang auf ihr nach hinten rutscht. Die Position der Kolbenstange 215 bei erstem Anschlag des Anschlagelements 241 an den Rand der proximalen Öffnung 217 des Injektorgehäuses wird zweite Vorschubposition genannt. Diese Position ist visuell gut erkennbar und kann je nach Stärke einer Klemmwirkung des Anschlagelements 241 auf dem Schaft der Kolbenstange oder bei vorhandener Quernut auf der Kolbenstange 215 auch taktil wahrgenommen werden, dadurch dass ein weiteres Vorschieben aufgrund eines höheren Widerstands (aufgrund der Reibung des Anschlagelements auf der Kolbenstange oder zumindest vorübergehend bei Überwinden der Quernut) spürbar ist.

Um die Kolbenstange 215 aus der zweiten Vorschubposition weiter in den Gang des Injektorgehäuses 213 zu schieben wird die Kolbenstange 215 durch das verschiebbare Anschlagelement 241, welches am Rand der proximalen Öffnung 217 des Injektorgehäuses 213 aufgehalten ist, hindurch eingeschoben. Sobald der Rampenkeil 245 am Injektorgehäuse 213, insbesondere am Rand der proximalen Öffnung 217 des Injektorgehäuses 213, anstösst, ist die dritte Vorstossposition der Kolbenstange 215 erreicht. Ist die Linse in dieser dritten Vorstossposition noch nicht ganz ausgestossen, können durch zusätzlichen Druck auf das Betätigungselement224 (z.B. mindestens 3 Newton, vorzugsweise mindestens 5 Newton) Rampenkeil 245 und Injektorgehäuses 213 gegeneinander gedrückt werden, wodurch gegebenenfalls durch Verformung des Injektorgehäuserands an der proximalen Öffnung 217 und/oder des Rampenkeils 245 die Kolbenstange 215 noch etwas weiter (z.B. bis zu ein paar Millimeter) vorrückt und die Linse ausstösst.

Es wurde hier die Anwendung eines verschiebbaren Anschlagelements (am Beispiel eines im Wesentlichen inkompressiblen Schlittens) in Kombination mit einer Rampe beschrieben.

### VERGLEICH DER ANWENDUNGSBEISPIELE 2 UND 3

Der im Anwendungsbeispiel 2 verwendete Soft-Anschlag steht im Gegensatz zum im Anwendungsbeispiel 3 verwendeten harten Anschlag, welcher aus einem Material besteht, das unter manuell aufgebrachter Kraft im Wesentlichen keine Deformation bzw. Kompression und somit an sich im Wesentlichen auch keinen zusätzlichen Vorschub zulässt. Im Ausführungsbeispiel 3 wird ein sogenannt harter Anschlag mit einer Rampe kombiniert. Sind Rampe und harter Anschlag derart aufeinander abgestimmt, dass unter erhöhtem Kraftaufwand der harte Anschlag sich unter zunehmender Deformation auf die Rampe stossen lässt, ergibt sich daraus ebenfalls ein Reservevorschub, welcher dazu genutzt werden kann, bei zu kurz ausgebildeter Kolbenstange die Linse unter zusätzlichem Kraftaufwand endgültig auszustossen.

Die Erfindung ist in den Ansprüchen definiert.

### BEZUGSZEICHENLISTE:

- 11: Injektor
- 13: Injektorgehäuse bzw. Injektorkörper
- 15: Kolbenstange, mit (a) vorderem Schaftbereich und (b) hinterem Schaftbereich
- 16: Kolbengang bzw. Injektorgang
- 17: proximales Ende des Injektorgehäuses
- 19: distales Ende des Injektorgehäuses
- 20: Stempel (auch Stössel genannt)
- 21: Aussparung
- 22: Ladevorrichtung
- 23: distales Ende der Kolbenstange, Kolbenstangenspitze
- 24: proximales Ende der Kolbenstange, Betätigungselement
- 25: (innerer) Griffbereich des ersten Flügels
- 26: (innerer) Griffbereich des zweiten Flügels
- 27: erster Flügel (auch Flügelgriff genannt)
- 28: zweiter Flügel (auch Flügelgriff genannt)
- 29: erster Gewindesteg
- 30: zweiter Gewindesteg
- 31: erste bewegliche Halterung
- 32: zweite bewegliche Halterung
- 33: Gewinde, bzw. Schaft mit Gewinde
- 34: Führung mit Kugelpfanne
- 35: Drehachse des ersten Flügelgriffs
- 36: Drehachse des zweiten Flügelgriffs
- 61: innerer Schenkel der ersten beweglichen Halterung
- 62: innerer Schenkel der zweiten beweglichen Halterung
- 71: äusserer Schenkel der ersten beweglichen Halterung
- 72: äusserer Schenkel der zweiten beweglichen Halterung
- 81: Exzenter (Druckmittel) des ersten Flügels
- 82: Exzenter (Druckmittel) des zweiten Flügels
- 83: erstes Achselement des ersten Flügels
- 84: erstes Achselement des zweiten Flügels
- 85: zweites Achselement des ersten Flügels
- 86: zweites Achselement des zweiten Flügels
- 89: Aussparung am Flügel
- 91: Teilzahnrad des ersten Flügels
- 92: Teilzahnrad des zweiten Flügels
- 111: Injektor
- 113: Injektorgehäuse bzw. Injektorkörper
- 115: Kolbenstange
- 116: Kolbengang bzw. Injektorgang
- 117: proximales Ende des Injektorgehäuses
- 119: distales Ende des Injektorgehäuses
- 121: Aussparung
- 123: distales Ende der Kolbenstange, Kolbenstangenspitze
- 124: proximales Ende der Kolbenstange, Betätigungselement
- 125: Griffbereich des ersten Flügels (Griffläche mit Rippenstruktur)
- 126: Griffbereich des zweiten Flügels (Griffläche mit Rippenstruktur)
- 124: erster Flügel (auch Flügelgriff genannt)
- 128: zweiter Flügel (auch Flügelgriff genannt)
- 133: Gewinde, bzw. Schaft mit Gewinde
- 135: Drehachse des ersten Flügelgriffs
- 136: Drehachse des zweiten Flügelgriffs
- 141: Anschlagelement
- 211: Injektor bzw. Injektorkörper
- 213: Injektorgehäuse
- 215: Kolbenstange
- 216: Kolbengang bzw. Injektorgang
- 217: proximales Ende des Injektorgehäuses
- 219: distales Ende des Injektorgehäuses
- 221: Aussparung
- 223: distales Ende der Kolbenstange, Kolbenstangenspitze
- 224: proximales Ende der Kolbenstange, Betätigungselement
- 227: erster Flügel (auch Flügelgriff genannt)
- 228: zweiter Flügel (auch Flügelgriff genannt)
- 233: Schaft mit Gleitbereich für ein Anschlagelement
- 241: Anschlagelement (im Wesentlichen inkompressibel)
- 245: Rampe, insb. keilartig ausgeführt
- 251: Federcliparretierung

## Patentansprüche

1. Injektor (11), insbesondere geeignet zum Injizieren einer intraokularen Linse in ein Auge oder zum Implantieren eines cornealen Endothelgewebes in ein Auge, beinhaltend einen länglichen Injektorkörper (13) mit Kolbengang (16), in welchem eine Injektorkolbenstange (15), welche ein Schraubgewinde (33) aufweist, longitudinal verschiebbar geführt ist, wobei der Injektor (11) zur Verschiebung der Injektorkolbenstange (15) mit zwei Betriebsmodi ausgestattet ist, zwischen welchen Modi umgeschaltet werden kann, wobei der erste Betriebsmodus einen Stossbetrieb und der zweite Betriebsmodus einen Schraubbetrieb definiert, **dadurch gekennzeichnet, dass**
der Injektorkörper (13) zumindest einen ein- und ausklappbaren Flügelgriff (27, 28) aufweist, wobei durch die ausgeklappte Position der Betriebsmodus auf Stossbetrieb und durch die eingeklappte Position der Betriebsmodus auf Schraubbetrieb gesetzt ist.

2. Injektor nach Anspruch 1, wobei der mindestens eine ein- und ausklappbare Flügelgriff (27, 28) in Bezug auf den Injektorkörper (13) von vorne her ausklappbar ist und nach vorne hin einklappbar ist.

3. Injektor nach einem der Ansprüche 1 oder 2, wobei der Injektorkörper (13) zumindest einen verschieblichen Gewindesteg (29, 30), insbesondere einen ein- und ausschiebbaren Gewindesteg (29, 30), aufweist.

4. Injektor nach einem der Ansprüche 3, wobei der Gewindesteg (29, 30) beim Ausklappen des Flügelgriffs (27, 28) aus dem Kolbengang (16) ausgeschoben und beim Einklappen des Flügelgriffs (27, 28) in den Kolbengang (16) eingeschoben wird.

5. Injektor nach einem der Ansprüche 3 oder 4, wobei der zumindest eine Flügelgriff (27, 28) und der zumindest eine Gewindesteg (29, 30) derart in Wirkverbindung angeordnet sind, sodass durch Einklappen des zumindest einen Flügelgriffs (27, 28) der zumindest eine Gewindesteg (29, 30) in eine erste Position führbar ist, in welcher der zumindest eine Gewindesteg (29, 30) ein Gegengewinde für das Schraubgewinde (33) der Injektorkolbenstange (15) bildet und durch Ausklappen des zumindest einen Flügelgriffs (27, 28) der zumindest eine Gewindesteg (29, 30) in eine zweite Position führbar ist, in welcher der zumindest eine Gewindesteg (29, 30) kein Gegengewinde für das Schraubgewinde (33) der Injektorkolbenstange (15) bildet.

6. Injektor nach einem der Ansprüche 3 bis 5, wobei Flügelgriff (27, 28) und Gewindesteg (29, 30) derart miteinander in Wirkverbindung stehen, dass bei eingeklapptem Flügelgriff (27, 28) durch mechanischen Druck der Kolbenstange (15) auf den Gewindesteg (29, 30), wie er beim Einschrauben der Kolbenstange entsteht, der Flügelgriff (27, 28) nicht aufklappen kann.

7. Injektor nach einem der Ansprüche 3 bis 6, wobei Flügelgriff (27, 28) und Gewindesteg (29, 30) derart miteinander in Wirkverbindung stehen, dass bei eingeklapptem Flügelgriff der Flügelgriff auch dann in eingeklappter Position bleibt, wenn beim Einschrauben der Kolbenstange die Kolbenstange mechanischen Druck auf den Gewindesteg ausübt.

8. Injektor nach einem der vorhergehenden Ansprüche 3 bis 7, wobei der zumindest eine ein- und ausklappbare Flügelgriff (27, 28) eine Drehachse (35, 36) aufweist, um welche der Flügelgriff (27, 28) ein- und ausklappbar ist.

9. Injektor nach Anspruch 8, wobei Flügelgriff (27, 28) und Gewindesteg (29, 30) über ein Druckmittel (81, 82) miteinander in Wirkverbindung stehen, welches am Flügelgriff (27, 28) exzentrisch zur Drehachse (35, 36) des Flügelgriffs (27, 28) ausgebildet ist.

10. Injektor nach Anspruch 9, wobei das Druckmittel (81, 82) bei eingeklapptem Flügelgriff (27, 28) den Gewindesteg (29, 30) in einer ersten Position hält, in welcher der zumindest eine Gewindesteg (29, 30) ein Gegengewinde für das Schraubgewinde (33) der Injektorkolbenstange (15) bildet, sodass die Injektorkolbenstange (15) schraubbar ist, und das Druckmittel (81, 82) bei ausgeklapptem Flügelgriff (27, 28) den Gewindesteg (29, 30) in einer zweiten Position hält, in welcher der zumindest eine Gewindesteg (29, 30) kein Gegengewinde für das Schraubgewinde (33) der Injektorkolbenstange (15) bildet, sodass die Injektorkolbenstange (15) stossbar ist.

11. Injektor nach einem der vorhergehenden Ansprüche 3 bis 10, wobei der zumindest eine Gewindesteg (29, 30) an zumindest einer beweglichen Halterung (31, 32) ausgebildet ist, welche derart beweglich ist, dass der Abstand des Gewindestegs (29, 30) zum Kolbengang (16) durch das Verstellen der Flügelgriffposition veränderbar ist.

12. Injektor nach Anspruch 11, wobei die zumindest eine bewegliche Halterung (31, 32) als Gabel mit einem inneren Schenkel (61, 62) und einem äusseren Schenkel (71, 72) ausgebildet ist, wobei bevorzugterweise die Schenkel das Druckmittel (81, 82) umfassen können.

13. Injektor nach einem der vorhergehenden Ansprüche 1 bis 12, wobei der zumindest eine Flügelgriff (27, 28) längsseitig am Injektorkörper (15) ein- und ausklappbar befestigt ist.

14. Injektor nach einem der vorhergehenden Ansprüche 1 bis 13, wobei
- der mindestens eine ein- und ausklappbare Flügelgriff (27, 28) als Doppelflügelgriff ausgeführt ist, mit einem ersten Flügel (27) und einem zweiten Flügel (28) , vorzugsweise dass der erste Flügel (27) und der zweite Flügel (28) einander gegenüberliegend längsseitig am Injektorkörper (13) ein- und ausklappbar befestigt sind, und/oder
- die Ein- und Ausklappbewegungen zweier Flügel (27, 28) synchronisiert sind, z.B. über eine Verzahnung (91, 92), und/oder
- der Flügelgriff (27, 28) derart ausgeführt ist, dass das Ein- und das Ausklappen des Flügelgriffs (27, 28) wiederholbar ist.

15. Injektor nach einem der vorhergehenden Ansprüche 1 bis 14, wobei die Injektorkolbenstange (15) rückseitig mit einem Betätigungselement (24) ausgeführt ist, welches zur manuellen Bedienung des Kolbens, z.B. zum manuellen Drücken oder Drehen des Kolbens, dient.

16. Injektor nach einem der Ansprüche 1-15, wobei die Injektorkolbenstange (15, 115, 215) ein Anschlagelement (141, 241) beinhaltet, und vorzugsweise
- wobei das Anschlagelement (141, 241) ein verschiebbares Anschlagelement (141, 241) ist und/oder das Anschlagelement (141, 241) als komprimierbares Anschlagelement ausgestaltet ist und/oder das Anschlagelement (141, 241) aus einem elastischen Material, insbesondere aus Silikon oder thermoplastischem Elastomer, gefertigt ist.

## Claims

1. An injector (11)in particular suitable for injecting an intraocular lens into an eye or for implanting a corneal endothelial tissue into an eye, comprising an elongate injector body (13) with piston passage (16), in which an injector piston rod (15) having a screw thread (33) is guided in a longitudinally displaceable manner, wherein the injector (11) is provided with two operating modes for the displacement of the injector piston rod (15), and is able to be switched between said modes, wherein the first operating mode defines a pushing operation and the second operating mode defines a screwing operation, **characterized in that**
the injector body (13) has at least one retractable and deployable wing grip (27, 28), wherein the operating mode is set to pushing operation by the deployed position and the operating mode is set to screwing operation by the retracted position.

2. The injector according to claim 1, wherein the at least one retractable and deployable wing grip (27, 28) can be deployed from the front and can be retracted towards the front with respect to the injector body (13).

3. The injector according to any one of claims 1 or 2, wherein the injector body (13) has at least one displaceable thread web (29, 30), in particular a thread web (29, 30) that can be pushed in and out.

4. The injector according to claim 3, wherein the thread web (29, 30) is pushed out of the piston passage (16) when the wing grip (27, 28) is deployed and pushed into the piston passage (16) when the wing grip (27, 28) is retracted.

5. The injector according to any one of claims 3 or 4, wherein the at least one wing grip (27, 28) and the at least one thread web (29, 30) are arranged in operative connection in such a way that by retracting the at least one wing grip (27, 28) the at least one thread web (29, 30) can be guided into a first position in which the at least one thread web (29, 30) forms a mating thread for the screw thread (33) of the injector piston rod (15) and by deploying the at least one wing grip (27, 28) the at least one thread web (29, 30) can be guided into a second position in which the at least one thread web (29, 30) does not form a mating thread for the screw thread (33) of the injector piston rod (15).

6. The injector according to any one of claims 3 to 5, wherein the wing grip (27, 28) and the thread web (29, 30) are in operative connection with one another in such a way that when the wing grip (27, 28) is retracted, the wing grip (27, 28) cannot be deployed by mechanical pressure of the piston rod (15) on the thread web (29, 30) as it arises when screwing in the piston rod.

7. The injector according to any one of claims 3 to 6, wherein the wing grip (27, 28) and the thread web (29, 30) are in operative connection with one another in such a way that when the wing grip is retracted, the wing grip also remains in the retracted position when, during screwing in the piston rod, the piston rod exerts mechanical pressure on the thread web.

8. The injector according to any one of the preceding claims 3 to 7, wherein the at least one retractable and deployable wing grip (27, 28) has an axis of rotation (35, 36) about which the wing grip (27, 28) is retractable and deployable.

9. The injector according to claim 8, wherein the wing grip (27, 28) and the thread web (29, 30) are in operative connection with one another via a pressure means (81, 82) which, on the wing grip (27, 28), is formed in an eccentric manner with respect to the axis of rotation (35, 36) of the wing grip (27, 28) .

10. The injector according to claim 9, wherein the pressure means (81, 82) holds the thread web (29, 30) in a first position when the wing grip (27, 28) is retracted, in which position the at least one thread web (29, 30) forms a mating thread for the screw thread (33) of the injector piston rod (15) so that the injector piston rod (15) can be screwed, and the pressure means (81, 82) holds the thread web (29, 30) in a second position when the wing grip (27, 28) is deployed, in which position the at least one thread web (29, 30) does not form a mating thread for the screw thread (33) of the injector piston rod (15), so that the injector piston rod (15) can be pushed.

11. The injector according to any one of the preceding claims 3 to 10, wherein the at least one thread web (29, 30) is formed on at least one movable holder (31, 32) which is movable in such a manner that the distance between the thread web (29, 30) to the piston passage (16) can be changed by adjusting the wing grip position.

12. The injector according to claim 11, wherein the at least one movable holder (31, 32) is designed as a fork with an inner leg (61, 62) and an outer leg (71, 72), wherein the legs preferably can comprise the pressure means (81, 82).

13. The injector according to any one of the preceding claims 1 to 12, wherein the at least one wing grip (27, 28) is attached to the injector body (15) along its length in a retractable and deployable manner.

14. The injector according to any one of the preceding claims 1 to 13, wherein
- the at least one retractable and deployable wing grip (27, 28) is designed as a double wing grip, with a first wing (27) and a second wing (28), preferably in that the first wing (27) and the second wing (28) are attached opposite one another along the length of the injector body (13) in a retractable and deployable manner, and/or
- the retracting and deploying movements of two wings (27, 28) are synchronized, e.g., via a toothing (91, 92) and/or
- the wing grip (27, 28) is designed in such a way that the retracting and deploying of the wing grip (27, 28) can be repeated.

15. The injector according to any one of the preceding claims 1 to 14, wherein the injector piston rod (15) is designed at the rear with an actuating element (24) which is used for manual operation of the piston, e.g., for manually pressing or turning the piston.

16. An injector according to any one of claims 1 to 15, wherein the injector piston rod (15, 115, 215) includes a stop element (141, 241), and preferably wherein the stop element (141, 241) is a displaceable stop element (141, 241) and/or the stop element (141, 241) is configured as a compressible stop element and/or the stop element (141, 241) is made from an elastic material, in particular from silicone or thermoplastic elastomer.

## Revendications

1. Injecteur (11), en particulier approprié pour injecter une lentille intraoculaire dans un oeil ou pour implanter un tissu endothélial cornéen dans un oeil, qui contient un corps d'injecteur allongé (13) avec un couloir de piston (16) dans lequel une tige de piston d'injecteur (15) qui présente un filetage (33) est guidée en étant déplaçable longitudinalement, cependant que l'injecteur (11) est équipé, pour le déplacement de la tige de piston d'injecteur (15), de deux modes de fonctionnement, modes entre lesquels il peut y avoir une commutation, le premier mode de fonctionnement définissant un mode de poussée et le second mode de fonctionnement un mode de vissage, **caractérisé en ce que**
le corps d'injecteur (13) présente au moins une manette à ailette qui peut être déployée et rabattue (27, 28), cependant que, par la position déployée, le mode de fonctionnement est mis sur mode de poussée et, par la position rabattue, le mode est mis sur mode de vissage.

2. Injecteur selon la revendication 1, cependant que la au moins une manette à ailette qui peut être déployée et rabattue (27, 28) peut, par rapport au corps d'injecteur (13), être déployée de l'avant et être rabattue vers l'avant.

3. Injecteur selon l'une des revendications 1 ou 2, cependant que le corps d'injecteur (13) présente au moins une âme filetée (29, 30) qui peut entrer et sortir par coulissement.

4. Injecteur selon la revendication 3, l'âme filetée (29, 30) sortant du couloir de piston (16) par coulissement lors du déploiement de la manette à ailette (27, 28) et rentrant dans le couloir de piston (16) lors du rabattement de la manette à ailette (27, 28).

5. Injecteur selon l'une des revendications 3 ou 4, cependant que la au moins une manette à ailette (27, 28) et la au moins une âme filetée (29, 30) sont placées en relation opérationnelle de telle manière que, lors du rabattement de la au moins une manette à ailette (27, 28), la au moins une âme filetée (29, 30) peut être guidée dans une première position dans laquelle la au moins une âme filetée (29, 30) forme un filet antagoniste pour le filetage (33) de la tige de piston d'injecteur (15) et, par le déploiement de la au moins une manette à ailette (27, 28), la au moins une âme filetée (29, 30) peut être guidée dans une seconde position dans laquelle la au moins une âme filetée (29, 30) ne forme pas de filet antagoniste pour le filetage (33) de la tige de piston d'injecteur (15).

6. Injecteur selon l'une des revendications 3 à 5, cependant que la manette à ailette (27, 28) et l'âme filetée (29, 30) sont placées en relation opérationnelle de telle manière que, lorsque la manette à ailette (27, 28) est rabattue, par la pression mécanique de la tige de piston (15) sur l'âme filetée (29, 30), telle qu'elle intervient lors du vissage de la tige de piston, la manette à ailette (27, 28) ne peut pas se déployer.

7. Injecteur selon l'une des revendications 3 à 6, cependant que la manette à ailette (27, 28) et l'âme filetée (29, 30) sont placées en relation opérationnelle de telle manière que, lorsque la manette à ailette (27, 28) est rabattue, la manette à ailette reste en position rabattue même lorsque, lors du vissage de la tige de piston, la tige de piston exerce une pression mécanique sur l'âme filetée.

8. Injecteur selon l'une des revendications 3 à 7, cependant que la au moins une manette à ailette qui peut être déployée et rabattue (27, 28) présente un axe de rotation (35, 36) autour duquel la manette à ailette (27, 28) peut être déployée et rabattue.

9. Injecteur selon la revendication 8, cependant que la manette à ailette (27, 28) et l'âme filetée (29, 30) sont en relation opérationnelle l'une avec l'autre par un moyen de pression (81, 82) qui est configuré sur la manette à ailette (27, 28) de manière excentrée par rapport à l'axe de rotation (35, 36) de la manette à ailette (27, 28).

10. Injecteur selon la revendication 9, cependant que le moyen de pression (81, 82) maintient, lorsque la manette à ailette (27, 28) est rabattue, l'âme filetée (29, 30) dans une première position dans laquelle la au moins une âme filetée (29, 30) forme un filet antagoniste pour le filetage (33) de la tige de piston d'injecteur (15) si bien que a tige de piston d'injecteur (15) peut être visée et le moyen de pression (81, 82) maintient, lorsque la manette à ailette (27, 28) est déployée, l'âme filetée (29, 30) dans une seconde position dans laquelle la au moins une âme filetée (29, 30) ne forme pas de filet antagoniste pour le filetage (33) de la tige de piston d'injecteur (15) si bien que la tige de piston d'injecteur (15) peut être poussée.

11. Injecteur selon l'une des revendications précédentes 3 à 10, cependant que la au moins une âme filetée (29, 30) est configurée sur au moins un support mobile (31, 32) qui est mobile de telle manière que la distance entre l'âme filetée (29, 30) et le couloir de piston (16) peut être varié par le changement de la position de la manette à ailette.

12. lnjecteur selon la revendication 11, cependant que le au moins un support mobile (31, 32) est configuré comme une fourche avec une branche intérieure (61, 62) et une branche extérieure (71, 72), cependant que de préférence le les montants peuvent entourer le moyen de pression (81, 82).

13. Injecteur selon l'une des revendications précédentes 1 à 12, cependant que la au moins une manette à ailette (27, 28) est fixée sur le côté longitudinal sur le corps d'injecteur (13) de manière à pouvoir être déployée et rabattue.

14. Injecteur selon l'une des revendications précédentes 1 à 13, cependant que
- la au moins une manette à ailette qui peut être déployée et rabattue (27, 28) est réalisée comme une manette à double ailette, avec une première ailette (27) et une seconde ailette (28), de préférence de telle manière que la première ailette (27) et la seconde ailette (28) sont fixées en face l'une de l'autre sur le côté longitudinal sur le corps d'injecteur (13) de manière à pouvoir être déployées et rabattues et/ou
- les mouvements de rabattement et de déploiement des deux ailettes (27, 28) sont synchronisés, par exemple par une denture (91, 92) et/ou
- la manette à ailette (27, 28) est réalisée de telle manière que le rabattement et le déploiement de la manette à ailette (27, 28) peut être répété.

15. Injecteur selon l'une des revendications précédentes 1 à 13, cependant que la tige de piston d'injecteur (15) est réalisée sur le côté arrière avec un élément d'actionnement (24) qui sert à la commande manuelle du piston, par exemple pour pousser ou tourner manuellement le piston.

16. Injecteur selon l'une des revendications 1 à 15, cependant que la tige de piston d'injecteur (15) contient un élément de butée (141, 241) et de préférence cependant que l'élément de butée (141, 241) est un élément de butée déplaçable (141, 241) et/ou que l'élément de butée (141, 241) est configuré comme un élément de butée compressible et/ou que l'élément de butée (141, 241) est fabriqué en un matériau élastique, en particulier en silicone ou en élastomère thermoplastique.
